# EUROPEAN PATENT APPLICATION

(11) **EP 3 034 074 A1**
(43) Date of publication of application: **22.06.2016**
(21) Application number: 14198823.8
(22) Date of filing: 18.12.2014
(51) Int. Cl.: A61K 31/198, A61K 31/4439, A61K 31/4709, A61K 31/4745, A61P 21/00

(54) **Compound for treatment of myotonic dystrophy type 1**

(71) Applicant: Universitat De València, Estudi General, 46010 València (ES)
(72) Inventor: Artero Allepuz, Rubén Darío, 46010 Valencia (ES); Bargiela Schönbrunn, Ariadna, 46010 Valencia (ES); Llamusí Troísi, Beatriz, 46010 Valencia (ES); Pérez Alonso, Manuel, 46010 Valencia (ES); Fernández Costa, Juan Manuel, 46010 Valencia (ES)
(74) Representative: Illescas, Manuel

(57) **Abstract**

The present invention relates to a compound, or a salt thereof, and a pharmaceutical composition comprising said compound, or a salt thereof, for use in the prevention and/or treatment of myotonic dystrophy type 1 in a patient, in particular by reducing the levels of autophagy and apoptosis that are elevated in myotonic muscular dystrophy type 1.

Sequence Listing

<110> University of Valencia

<120> Compound for treatment of myotonic dystrophy type 1

<130> EP-06978

<160> 34

<170> BiSSAP 1.3

<210> 1
<211> 20
<212> DNA
<213> Artificial Sequence

<220>
<223> Forward primer

<400> 1
ggaaagtcct tggggtcttc                                                    20

<210> 2
<211> 20
<212> DNA
<213> Artificial Sequence

<220>
<223> Reverse primer

<400> 2
ggaactgatg aatgggagca                                                    20

<210> 3
<211> 20
<212> DNA
<213> Artificial Sequence

<220>
<223> Forward primer

<400> 3
caccgacgct aatgatgttg                                                    20

<210> 4
<211> 20
<212> DNA
<213> Artificial Sequence

<220>
<223> Reverse primer

<400> 4
tttgttttct gcctccactg                                                    20

<210> 5
<211> 21

<212> DNA
<213> Artificial Sequence

<220>
<223> Forward Primer

<400> 5
catagcctgt tcagcggccg t                                                21


<210> 6
<211> 25
<212> DNA
<213> Artificial Sequence

<220>
<223> Reverse Primer

<400> 6
ccgcttgaat tcggagattc ccgtc                                            25


<210> 7
<211> 22
<212> DNA
<213> Artificial Sequence

<220>
<223> Forward Primer

<400> 7
atccagaccg tgtgcccgtc at                                               22


<210> 8
<211> 21
<212> DNA
<213> Artificial Sequence

<220>
<223> Reverse Primer

<400> 8
accgacggtc aggtcggaag g                                                21


<210> 9
<211> 25
<212> DNA
<213> Artificial Sequence

<220>
<223> Forward Primer

<400> 9
tcgatgccag cgagcaaatt ttcct                                            25


<210> 10
<211> 21
<212> DNA
<213> Artificial Sequence

<220>
<223> Reverse Primer

<400> 10
gccccacgcc tgattcttgc a                                                21


<210> 11
<211> 21
<212> DNA
<213> Artificial Sequence

<220>
<223> Forward Primer

<400> 11
atgaccatcc gcccagcata c                                                21


<210> 12
<211> 21
<212> DNA
<213> Artificial Sequence

<220>
<223> Reverse Primer

<400> 12
atgtggcggg tgcgcttgtt c                                                21


<210> 13
<211> 20
<212> DNA
<213> Artificial Sequence

<220>
<223> Forward Primer

<400> 13
agcccacaga gacagagacc                                                  20


<210> 14
<211> 20
<212> DNA
<213> Artificial Sequence

<220>
<223> Reverse Primer

<400> 14
cgtcctcatc catcacaaag                                                  20


<210> 15
<211> 20
<212> DNA
<213> Artificial Sequence

<220>
<223> Forward Primer

<400> 15
ctcacacagt caccgagagc                                                    20


<210> 16
<211> 20
<212> DNA
<213> Artificial Sequence

<220>
<223> Reverse Primer

<400> 16
tgggtctgga aggcatactt                                                    20


<210> 17
<211> 20
<212> DNA
<213> Artificial Sequence

<220>
<223> Forward Primer

<400> 17
tttgctcaga tggatgttcg                                                    20


<210> 18
<211> 21
<212> DNA
<213> Artificial Sequence

<220>
<223> Reverse Primer

<400> 18
tcctcagctt gctggtacac t                                                  21


<210> 19
<211> 19
<212> DNA
<213> Artificial Sequence

<220>
<223> Forward Primer

<400> 19
cacctgtggt ccacctgac                                                     19


<210> 20
<211> 18
<212> DNA
<213> Artificial Sequence

<220>
<223> Reverse Primer

<400> 20
ctggacatct cggcgaag                                                      18

<210> 21
<211> 21
<212> DNA
<213> Artificial Sequence

<220>
<223> Forward Primer

<400> 21
ccggtcaaaa ggaagagact t                                                21


<210> 22
<211> 18
<212> DNA
<213> Artificial Sequence

<220>
<223> Reverse Primer

<400> 22
tgcgtcttcc ggttcttc                                                    18


<210> 23
<211> 19
<212> DNA
<213> Artificial Sequence

<220>
<223> Forward Primer

<400> 23
agccacatcg ctcagacac                                                   19


<210> 24
<211> 18
<212> DNA
<213> Artificial Sequence

<220>
<223> Reverse Primer

<400> 24
cgcccaatac gaccaaat                                                    18


<210> 25
<211> 20
<212> DNA
<213> Artificial Sequence

<220>
<223> Forward Primer

<400> 25
aatggcacag tgagcacaaa                                                  20


<210> 26
<211> 20

<212> DNA
<213> Artificial Sequence

<220>
<223> Reverse Primer

<400> 26
gagatggcac agtggtgtgt                                                    20


<210> 27
<211> 21
<212> DNA
<213> Artificial Sequence

<220>
<223> Forward Primer

<400> 27
tgctggaagc ctttgtctat g                                                  21


<210> 28
<211> 20
<212> DNA
<213> Artificial Sequence

<220>
<223> Reverse Primer

<400> 28
cgcttgttgc ctttggtatt                                                    20


<210> 29
<211> 18
<212> DNA
<213> Artificial Sequence

<220>
<223> Forward Primer

<400> 29
ggtggggttt tccctgag                                                      18


<210> 30
<211> 24
<212> DNA
<213> Artificial Sequence

<220>
<223> Reverse Primer

<400> 30
gaaattctga ggattcaggc tatg                                               24


<210> 31
<211> 16
<212> DNA
<213> Artificial Sequence

<220>
<223> Forward Primer

<400> 31
cggctccggg tcaagg                                                        16


<210> 32
<211> 24
<212> DNA
<213> Artificial Sequence

<220>
<223> Reverse Primer

<400> 32
cttctggagg ataaactctc ggat                                               24


<210> 33
<211> 19
<212> DNA
<213> Artificial Sequence

<220>
<223> Forward Primer

<400> 33
cagaaggcaa agctggaga                                                     19


<210> 34
<211> 21
<212> DNA
<213> Artificial Sequence

<220>
<223> Reverse Primer

<400> 34
tccacctgca gaaggtctaa c                                                  21

SEQUENCE LISTING

<110> Universitat De València, Estudi General

<120> Compound for treatment of myotonic dystrophy type 1

<130> EP-06978

<140> EP14198823.8
<141> 2014-12-18

<160> 35

<170> BiSSAP 1.3

<210> 1
<211> 20
<212> DNA
<213> Artificial Sequence

<220>
<223> Forward primer

<400> 1
ggaaagtcct tggggtcttc                                                    20


<210> 2
<211> 20
<212> DNA
<213> Artificial Sequence

<220>
<223> Reverse primer

<400> 2
ggaactgatg aatgggagca                                                    20


<210> 3
<211> 20
<212> DNA
<213> Artificial Sequence

<220>
<223> Forward primer

<400> 3
caccgacgct aatgatgttg                                                    20


<210> 4
<211> 20
<212> DNA
<213> Artificial Sequence

<220>
<223> Reverse primer

<400> 4
tttgttttct gcctccactg                                                    20

<210> 5
<211> 21
<212> DNA
<213> Artificial Sequence

<220>
<223> Forward primer

<400> 5
catagcctgt tcagcggccg t                                                21


<210> 6
<211> 25
<212> DNA
<213> Artificial Sequence

<220>
<223> Reverse Primer

<400> 6
ccgcttgaat tcggagattc ccgtc                                            25


<210> 7
<211> 22
<212> DNA
<213> Artificial Sequence

<220>
<223> Forward Primer

<400> 7
atccagaccg tgtgcccgtc at                                               22


<210> 8
<211> 21
<212> DNA
<213> Artificial Sequence

<220>
<223> Reverse Primer

<400> 8
accgacggtc aggtcggaag g                                                21


<210> 9
<211> 25
<212> DNA
<213> Artificial Sequence

<220>
<223> Forward Primer

<400> 9
tcgatgccag cgagcaaatt ttcct                                            25


<210> 10
<211> 21
<212> DNA

<213> Artificial Sequence

<220>
<223> Reverse Primer

<400> 10
gccccacgcc tgattcttgc a                                                21


<210> 11
<211> 21
<212> DNA
<213> Artificial Sequence

<220>
<223> Forward Primer

<400> 11
atgaccatcc gcccagcata c                                                21


<210> 12
<211> 21
<212> DNA
<213> Artificial Sequence

<220>
<223> Reverse primer

<400> 12
atgtggcggg tgcgcttgtt c                                                21


<210> 13
<211> 20
<212> DNA
<213> Artificial Sequence

<220>
<223> Forward Primer

<400> 13
agcccacaga gacagagacc                                                  20


<210> 14
<211> 20
<212> DNA
<213> Artificial Sequence

<220>
<223> Reverse Primer

<400> 14
cgtcctcatc catcacaaag                                                  20


<210> 15
<211> 20
<212> DNA
<213> Artificial Sequence

<220>

<223> Forward Primer

<400> 15
ctcacacagt caccgagagc                                                    20


<210> 16
<211> 20
<212> DNA
<213> Artificial Sequence

<220>
<223> Reverse Primer

<400> 16
tgggtctgga aggcatactt                                                    20


<210> 17
<211> 20
<212> DNA
<213> Artificial Sequence

<220>
<223> Forward Primer

<400> 17
tttgctcaga tggatgttcg                                                    20


<210> 18
<211> 21
<212> DNA
<213> Artificial Sequence

<220>
<223> Reverse Primer

<400> 18
tcctcagctt gctggtacac t                                                  21


<210> 19
<211> 19
<212> DNA
<213> Artificial Sequence

<220>
<223> Forward Primer

<400> 19
cacctgtggt ccacctgac                                                     19


<210> 20
<211> 18
<212> DNA
<213> Artificial Sequence

<220>
<223> Reverse Primer

<400> 20

ctggacatct cggcgaag                                                    18

<210> 21
<211> 21
<212> DNA
<213> Artificial Sequence

<220>
<223> Forward Primer

<400> 21
ccggtcaaaa ggaagagact t                                                21

<210> 22
<211> 18
<212> DNA
<213> Artificial Sequence

<220>
<223> Reverse Primer

<400> 22
tgcgtcttcc ggttcttc                                                    18

<210> 23
<211> 19
<212> DNA
<213> Artificial Sequence

<220>
<223> Forward Primer

<400> 23
agccacatcg ctcagacac                                                   19

<210> 24
<211> 18
<212> DNA
<213> Artificial Sequence

<220>
<223> Reverse Primer

<400> 24
cgcccaatac gaccaaat                                                    18

<210> 25
<211> 20
<212> DNA
<213> Artificial Sequence

<220>
<223> Forward Primer

<400> 25
aatggcacag tgagcacaaa                                                  20

<210> 26
<211> 20
<212> DNA
<213> Artificial Sequence

<220>
<223> Reverse Primer

<400> 26
gagatggcac agtggtgtgt                                                    20


<210> 27
<211> 21
<212> DNA
<213> Artificial Sequence

<220>
<223> Forward Primer

<400> 27
tgctggaagc ctttgtctat g                                                  21


<210> 28
<211> 20
<212> DNA
<213> Artificial Sequence

<220>
<223> Reverse Primer

<400> 28
cgcttgttgc ctttggtatt                                                    20


<210> 29
<211> 18
<212> DNA
<213> Artificial Sequence

<220>
<223> Forward Primer

<400> 29
ggtggggttt tccctgag                                                      18


<210> 30
<211> 24
<212> DNA
<213> Artificial Sequence

<220>
<223> Reverse Primer

<400> 30
gaaattctga ggattcaggc tatg                                               24


<210> 31
<211> 16
<212> DNA

<213> Artificial Sequence

<220>
<223> Forward Primer

<400> 31
cggctccggg tcaagg                                                    16


<210> 32
<211> 24
<212> DNA
<213> Artificial Sequence

<220>
<223> Reverse Primer

<400> 32
cttctggagg ataaactctc ggat                                           24


<210> 33
<211> 19
<212> DNA
<213> Artificial Sequence

<220>
<223> Forward Primer

<400> 33
cagaaggcaa agctggaga                                                 19


<210> 34
<211> 21
<212> DNA
<213> Artificial Sequence

<220>
<223> Reverse Primer

<400> 34
tccacctgca gaaggtctaa c                                              21


<210> 35
<211> 20
<212> DNA
<213> Artificial Sequence

<220>
<223> Probe

<400> 35
cagcagcagc agcagcagca                                                20

## Description

### Field of the Invention

The present invention relates to the provision of a compound or a salt thereof, for the prevention and/or treatment of myotonic dystrophy type 1 in a patient. Likewise, the present invention additionally relates to the use of said composition or a salt thereof in the prevention and/or treatment of myotonic dystrophy type 1 in a patient. Moreover, the present invention also discloses a pharmaceutical composition for the prevention and/or treatment of myotonic dystrophy type 1, comprising the aforementioned composition or a salt thereof. Furthermore, the present invention also discloses a method for the prevention and/or treatment of myotonic dystrophy type 1 in a patient.

### Background to the Invention

Myotonic dystrophy type 1 (DM1; OMIM 160900, Steinert disease) is a myotonic muscular dystrophy and the most common form of muscular dystrophy in adults with a prevalence estimated at 1 in 8000. Characteristic symptoms are formation of iridescent cataracts, defects in cardiac conduction, endocrine changes, muscle weakness due to muscle mass wasting (known as atrophy) and muscle distress with myotonia, as well as defects in central nervous system function (1, 2). Of these, muscle mass wasting in adultsis one of the most debilitating symptoms of DM1 disease, ultimately leading to immobility, respiratory defects (respiratory distress), dysarthria, dysphagia and death in advanced stages of the disease. Besides symptomatic identification of DM1, this disease may also be identified by genetic tests, in particular DNA sequencing, southern blot and PCR methods. DM1 is a chronic, inherited multi-systemic disease with an autosomal dominant pattern of inheritance. In DM1, there is an expansion of the cytosine-thymine-guanine (CTG) triplet repeat in the 3' untranslated region of the *Dystrophia myotonica protein kinase* gene *(DMPK)* which is located on the long arm of chromosome 19. Said *DMPK* gene encodes myotonic dystrophy protein kinase which is expressed predominantly in skeletal muscle. The most abundant isoform of DMPK is an 80 kDa protein mainly expressed in smooth, skeletal and cardiac muscles. The presence of between 5 and 37 CTG triplet repeats in the *DMPK* gene is normal. However, individuals with between 38 and 49 triplet repeats are considered to have a pre-mutation and are at risk of having children with further expanded repeats and, therefore, symptomatic disease. Moreover, individuals with greater than 50 triplet repeats are almost invariably symptomatic DM1 patients. Thus, longer CTG triplet repeats are associated with earlier onset and more severe disease.

It is now well-established that non-coding CUG repeat expansions play a toxic gain-of-function role in DM1. Mutant RNAs containing expanded CUG repeats have increased stability and reduced turnover, which is likely responsible for the accumulation of non-degraded mutant RNA within patient cells. The accumulation of mutant RNA is toxic and alters gene expression at several levels, particularly alternative splicing, as has been demonstrated in different cell lines and animal models (3). However, muscle atrophy in adult tissues has not yet been directly linked to any mis-splicing event or other RNA metabolism alteration to date. The molecular mechanism of muscle atrophy in DM1 is, thus, poorly understood. MBNL1 is a splicing factor that is sequestered by CTG repeats forming ribonuclear foci. CELF1 is another splicing factor antagonistic to MBNL1 and its activity is increased upon hyperphosphorylation.

Autophagy is considered a pro-survival mechanism as it eliminates toxic proteins and damaged organelles in the cell. When this mechanism is up-regulated, however, protein homeostasis alteration and cell death is observed. Impaired apoptosis and autophagy levels have been reported in muscles of patients affected by Duchenne muscular dystrophy (DMD) as well as in the *mdx* mouse model (4, 5). In fact, in *mdx* mice, as well as in a mouse model with defective collagen VI, it was reported that the modulation of autophagic levels by a low protein diet could represent a therapeutic approach to these diseases. In addition, muscular dystrophies linked to collagen VI deficiency show dysfunctional mitochondria and spontaneous apoptosis caused by defective autophagy (6, 7). The role of autophagy and ubiquitin-proteasome in the pathogenesis of myopathies and muscular dystrophies has also been reported (8).

The use of autophagy inhibitors in the treatment of diseases including merosin (laminin ct2) deficient congenital muscular dystrophy (MDC1A) and collagen VI deficient CMD has been reported (9). In addition, compositions that may comprise mixtures of antiapoptotic agents with other agents have been proposed for use against neurodegenerative disorders, including trinucleotide repeat expansion disorders (10, 11). However, because the aforementioned disorders and diseases are a heterogeneous group, there is no evidence that the reported treatments may also be effective against any other particular type of muscular dystrophy, let alone evidence showing the extent of their effectiveness.

It is a problem of the present invention to provide a compound for the prevention and/or treatment of myotonic dystrophy type 1, which restores the phenotype of a patient with said disease or condition to a non-diseased state. Moreover, it is a problem of the present invention to provide a compound for the improved prevention and/or treatment of myotonic dystrophy type 1, in a patient with said disease.

In addition it is a problem of the present invention to provide a compound which may be administered by a non-invasive means.

### Brief Description of the Invention

The present invention relates to a compound, or a salt thereof, for use in the prevention and/or treatment of myotonic dystrophy type 1 in a patient, wherein said compound is selected from:
a) a compound selected from the formula 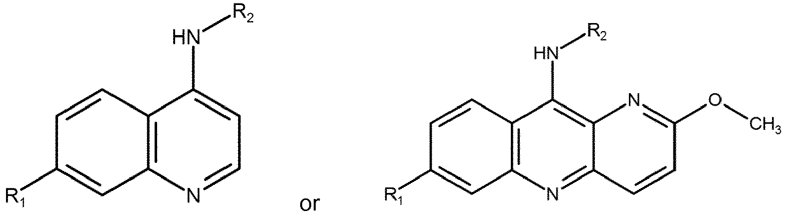 whereby
   R₁ is selected from H or a halide; and
   R₂ is selected from an alkyl moiety substituted with a moiety selected from an amino, alkyl amino, dialkyl amino, cycloamino or hydroxyalkyl amino moiety or a phenolic moiety substituted with at least one moiety selected from a aminoalkyl, (alkylamino)alkyl, (dialkylamino)alkyl, (cycloamino)alkyl or (hydroxyalkylamino)alkyl moiety;
b) a compound of the formula R³SCH₂-CH(NHR⁴)-COR⁵, wherein
   R³ is selected from H, -CH₂CH₂CH₂OH or -CH₂COOH;
   R⁴ is selected from H or -C(=O)CH₃; and
   R⁵ is selected from -OH, -OCH₃ or -NH₂; and
   whereby R³ and R⁴ cannot both be H when R⁵ is -OH; or
c) a compound of the formula 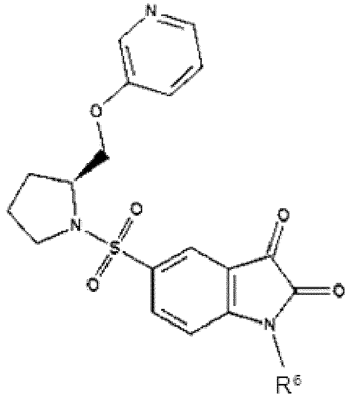 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties.

Analogously, the present invention also relates to use of a compound in the manufacture of a medicament for the prevention and/or treatment of myotonic dystrophy type 1, wherein said compound is selected from:
a) a compound selected from the formula 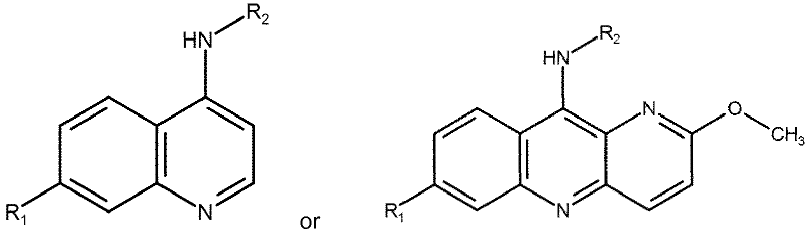 whereby
   R₁ is selected from H or a halide; and
   R₂ is selected from an alkyl moiety substituted with a moiety selected from an amino, alkyl amino, dialkyl amino, cycloamino or hydroxyalkyl amino moiety or a phenolic moiety substituted with at least one moiety selected from a aminoalkyl, (alkylamino)alkyl, (dialkylamino)alkyl, (cycloamino)alkyl or (hydroxyalkylamino)alkyl moiety;
b) a compound of the formula R³SCH₂-CH(NHR⁴)-COR⁵, wherein
   R³ is selected from H, -CH₂CH₂CH₂OH or -CH₂COOH;
   R⁴ is selected from H or -C(=O)CH₃; and
   R⁵ is selected from -OH, -OCH₃ or -NH₂; and
   whereby R³ and R⁴ cannot both be H when R⁵ is -OH; or
c) a compound of the formula 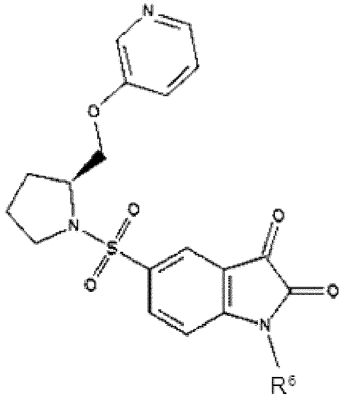 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties.

In addition, the present invention relates to a pharmaceutical composition for use in the prevention and/or treatment of myotonic dystrophy type 1 in a patient comprising:
I) a compound, or a salt thereof, selected from
   a) a compound selected from the formula 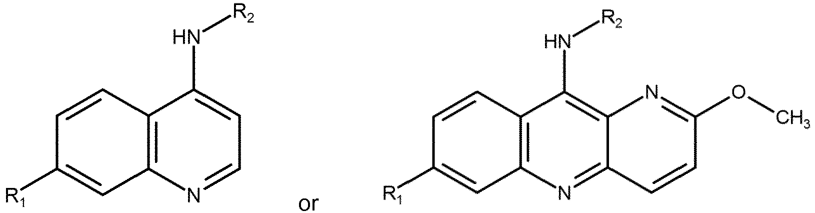 whereby
      R₁ is selected from H or a halide; and
      R₂ is selected from an alkyl moiety substituted with a moiety selected from an amino, alkyl amino, dialkyl amino, cycloamino or hydroxyalkyl amino moiety; or a phenolic moiety substituted with at least one moiety selected from a aminoalkyl, (alkylamino)alkyl, (dialkylamino)alkyl, (cycloamino)alkyl or (hydroxyalkylamino)alkyl moiety;
   b) a compound of the formula R³SCH₂-CH(NHR⁴)-COR⁵, wherein
      R³ is selected from H, -CH₂CH₂CH₂OH or -CH₂COOH;
      R⁴ is selected from H or -C(=O)CH₃; and
      R⁵ is selected from -OH, -OCH₃ or -NH₂; and
      whereby R³ and R⁴ cannot both be H when R⁵ is -OH; or
   c) a compound of the formula 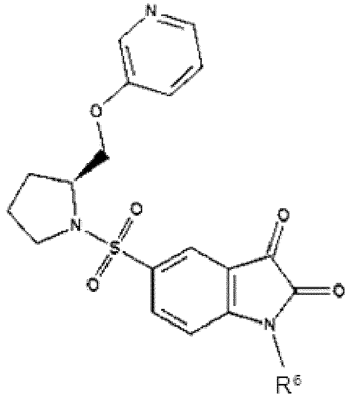 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties; and
II) at least one excipient and/or carrier.

Analogously, the present invention relates to use of a pharmaceutical composition in the manufacture of a medicament for the prevention and/or treatment of myotonic dystrophy type 1 in a patient, wherein said pharmaceutic composition comprises:
I) a compound, or a salt thereof, selected from
   a) a compound selected from the formula 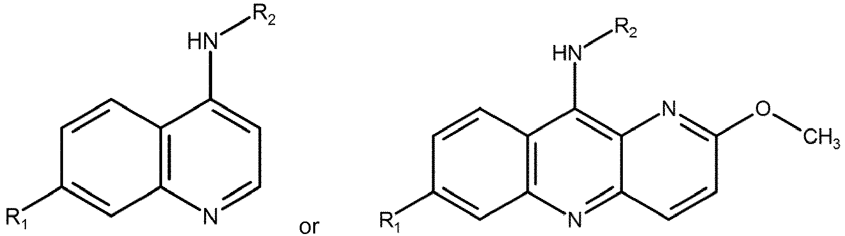 whereby
      R₁ is selected from H or a halide; and
      R₂ is selected from an alkyl moiety substituted with a moiety selected from an amino, alkyl amino, dialkyl amino, cycloamino or hydroxyalkyl amino moiety; or a phenolic moiety substituted with at least one moiety selected from a aminoalkyl, (alkylamino)alkyl, (dialkylamino)alkyl, (cycloamino)alkyl or (hydroxyalkylamino)alkyl moiety;
   b) a compound of the formula R³SCH₂-CH(NHR⁴)-COR⁵, wherein
      R³ is selected from H, -CH₂CH₂CH₂OH or -CH₂COOH;
      R⁴ is selected from H or -C(=O)CH₃; and
      R⁵ is selected from -OH, -OCH₃ or -NH₂; and
      whereby R³ and R⁴ cannot both be H when R⁵ is -OH; or
   c) a compound of the formula 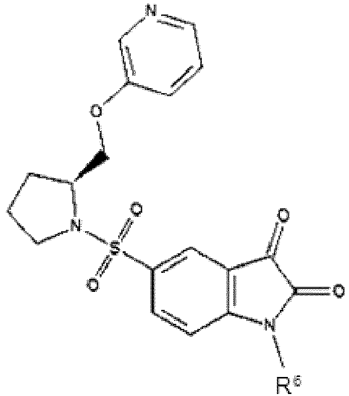 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties; and
II) at least one excipient and/or carrier.

Moreover, the present invention relates to a method for producing a pharmaceutical composition, which comprises mixing:
I) a compound, or a salt thereof, of the formula 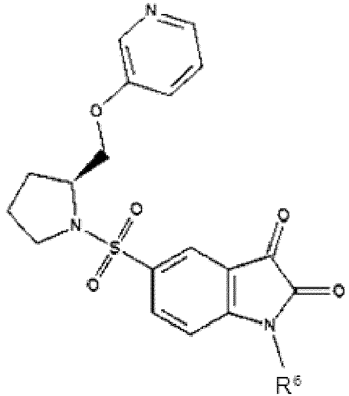 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties; and
II) at least one excipient and/or carrier.

Furthermore, the present invention also discloses a kit of parts comprising:
i) a pharmaceutical composition comprising:
   a compound, or a salt thereof, of the formula 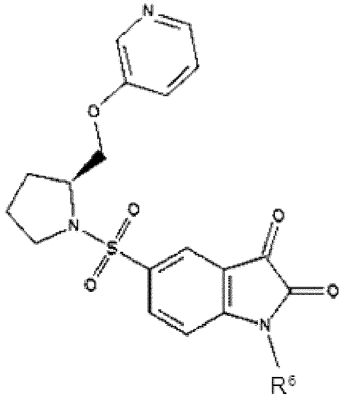 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties, and
      at least one excipient and/or carrier; and
ii) a container,
   wherein said pharmaceutical composition is contained in said container.

In addition, the present invention relates to a method of prevention and/or treatment of myotonic dystrophy type 1 in a patient, said method comprising administering to said patient a compound, a salt thereof or a pharmaceutical composition comprising the same in pharmaceutically effective dose, according to a continuous schedule having a dosing interval selected from the group consisting of continuous dosing, once-daily dosing, twice-daily dosing, thrice-daily dosing, four-times daily dosing, five-times daily dosing, six-times daily dosing, seven-times daily dosing, once-weekly dosing, twice-weekly dosing, thrice-weekly, four-times weekly, five-times weekly, six-times weekly, biweekly dosing and twice-monthly dosing, wherein said compound is selected from
a) a compound selected from the formula 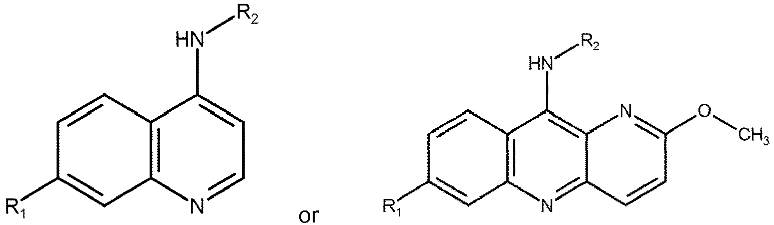 whereby
   R₁ is selected from H or a halide; and
   R₂ is selected from an alkyl moiety substituted with a moiety selected from an amino, alkyl amino, dialkyl amino, cycloamino or hydroxyalkyl amino moiety or a phenolic moiety substituted with at least one moiety selected from a aminoalkyl, (alkylamino)alkyl, (dialkylamino)alkyl, (cycloamino)alkyl or (hydroxyalkylamino)alkyl moiety;
b) a compound of the formula R³SCH₂-CH(NHR⁴)-COR⁵, wherein
   R³ is selected from H, -CH₂CH₂CH₂OH or -CH₂COOH;
   R⁴ is selected from H or -C(=O)CH₃; and
   R⁵ is selected from -OH, -OCH₃ or -NH₂; and
   whereby R³ and R⁴ cannot both be H when R⁵ is -OH; or
c) a compound of the formula 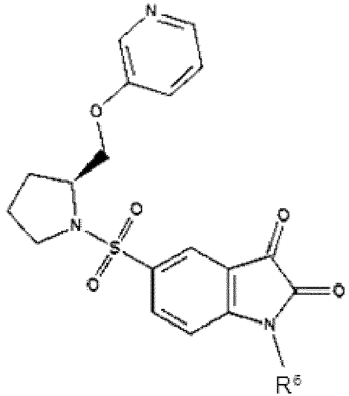 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties.

Preferably, administration is carried out so as to achieve an improvement in at least one symptom associated with myotonic dystrophy type 1 in a patient by at least 60%.

The present invention additionally discloses a compound for use in the prevention and/or treatment of myotonic dystrophy type 1 in a human patient, wherein said compound is selected from chloroquine, chloroquine phosphate, chloroquine sulfate, chloroquine hydrochloride, *N*-acetyl L-cysteine, *N*-acetyl L-cysteine amide, or 1-(4-methoxybenzyl)-5-({(2S)-2-[(3-pyridinyloxy)methyl]-1-pyrrolidinyl}sulfonyl)-1*H*-indole-2,3-dione.

Analogously, the present invention also relates to use of a compound in the manufacture of a medicament for the prevention and/or treatment of myotonic dystrophy type 1 in a human patient, wherein said compound is selected from chloroquine, chloroquine phosphate, chloroquine sulfate, chloroquine hydrochloride, *N*-acetyl L-cysteine, *N*-acetyl L-cysteine amide, or 1-(4-methoxybenzyl)-5-({(2*S*)-2-[(3-pyridinyloxy)methyl]-1-pyrrolidinyl}sulfonyl)-1*H*-indole-2,3-dione.

In addition, the present invention relates to a pharmaceutical composition for use in the prevention and/or treatment of myotonic dystrophy type 1 in a human patient comprising:
I) a compound, or a salt thereof, wherein said compound is selected from chloroquine, chloroquine phosphate, chloroquine sulfate, chloroquine hydrochloride, *N-*acetyl L-cysteine, *N*-acetyl L-cysteine amide, or 1-(4-methoxybenzyl)-5-({(2*S*)-2-[(3-pyridinyloxy)methyl]-1-pyrrolidinyl}sulfonyl)-1*H*-indole-2,3-dione; and
II) at least one excipient and/or carrier.

Moreover, the present invention relates to a method for producing a pharmaceutical composition, which comprises mixing:
I) 1-(4-methoxybenzyl)-5-({(2*S*)-2-[(3-pyridinyloxy)methyl]-1-pyrrolidinyl}sulfonyl)-1*H-*indole-2,3-dione or a salt thereof; and
II) at least one excipient and/or carrier.

Furthermore, the present invention also discloses a kit of parts comprising:
i) a pharmaceutical composition comprising 1-(4-methoxybenzyl)-5-({(2*S*)-2-[(3-pyridinyloxy)methyl]-1-pyrrolidinyl}sulfonyl)-1*H*-indole-2,3-dione or a salt thereof; and at least one excipient and/or carrier; and
ii) a container,
wherein said pharmaceutical composition is contained in said container.

In addition, the present invention relates to a method of prevention and/or treatment of myotonic dystrophy type 1 in a patient, said method comprising administering orally to said patient a compound, a salt thereof or a pharmaceutical composition comprising the same, according to a continuous schedule having a dosing interval selected from the group consisting of continuous dosing, once-weekly dosing, twice-weekly dosing, biweekly dosing and twice-monthly dosing, wherein said compound is selected from chloroquine, chloroquine phosphate, chloroquine sulfate, chloroquine hydrochloride, *N*-acetyl L-cysteine, *N*-acetyl L-cysteine amide, or 1-(4-methoxybenzyl)-5-({(2*S*)-2-[(3-pyridinyloxy)methyl]-1-pyrrolidinyl}sulfonyl)-1*H*-indole-2,3-dione. Preferably, administration is carried out so as to achieve an improvement in at least one symptom associated with myotonic dystrophy type 1 in a patient by at least 70%.

### Brief Description of the Figures

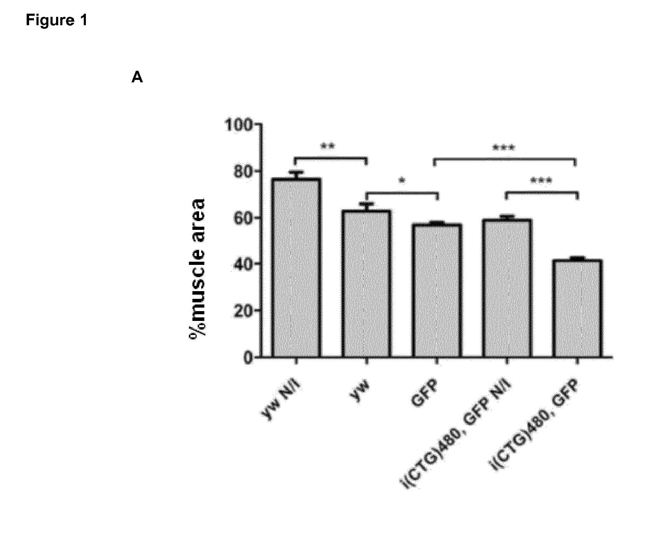

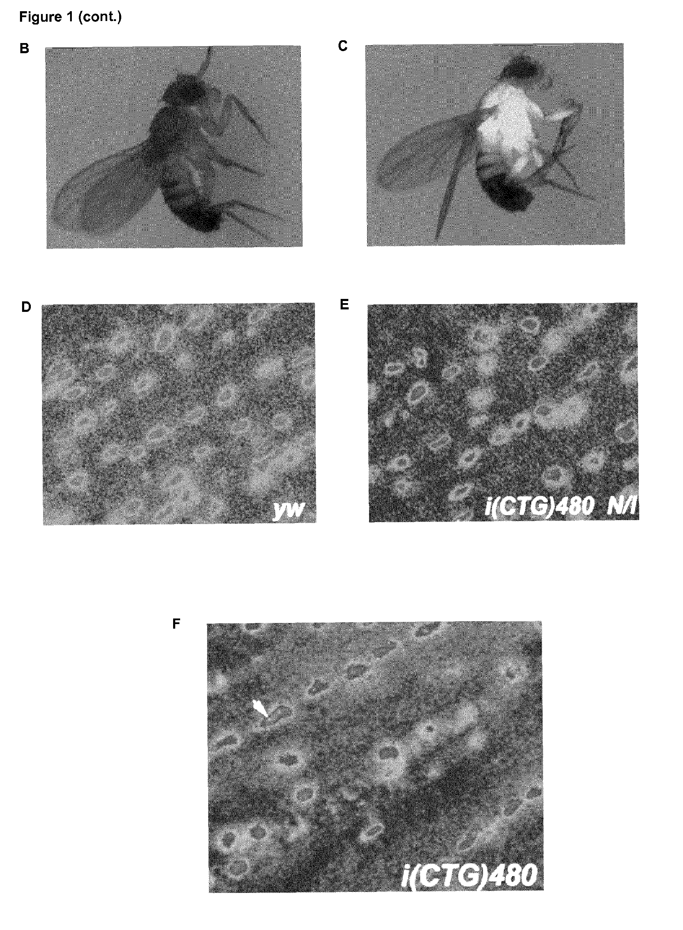

**Figure 1****.** Characterization of a *Drosophila* inducible model of DM1.
   **(A)** Quantification of the mean percentage of muscle area of dorsoventral sections of resin-embedded thoraces of flies of the indicated genotypes under the control of the *hs-*Gal4 driver. Statistically significant differences are denoted by asterisks (**P* < 0.05, ***P <* 0.01, ****P* < 0.001). The graph shows means ± s.e.m. Cross-sectional muscle area differences between IFM of flies that were or were not heat-shocked (uninduced = N/I) were evident (*cf*. yw N/I *vs. yw* and *i(CTG)480, GFP N*/*I vs. i(CTG)480, GFP).* Expression of GFP transgene also caused a reduction of muscle area (*cf*. *yw* and *hs-Gal4>UAS-GFP*). The indirect flight muscles (IFM) area in control fly thoraces *(cf. hs-Gal4>UAS-GFP*) was further reduced by the expression of expanded CTG repeat (*cf*. *hs-Gal4>UAS-i(CTG)480 UAS-GFP).* **(B, C)** Fluorescence dissecting microscope images of model flies (*hs-Gal4>UASi(CTG)480 UAS-GFP)* show strong GFP expression when transgene expression was induced (B) in comparison to uninduced counterparts (A). (D - F) Representative fluorescent *in situ* detection of *i(CTG)480* transcripts in longitudinal cryosections of IFM of *yw* (D) or *hs-Gal4>UAS-i(CTG)480* uninduced (E) or induced flies (F). Ribonuclear aggregates (exemplified by arrow head) were only detected in model flies after heat-shock treatment. Nuclei were counterstained with DAPI.

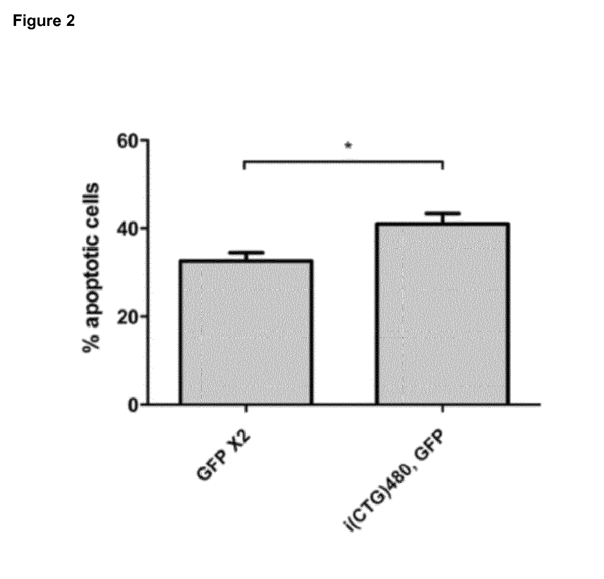

**Figure 2****.** Inducible DM1 model displays increased apoptosis.
   Quantification of apoptotic cells in representative fluorescent micrographs of longitudinal cryosections from adult *Drosophila* thoraces. Fragmented DNA was stained with TMR and nuclei were counterstained with DAPI. Increased level of apoptosis of 25% is observed in model flies *(hs-Gal4 UAS-i(CTG)480>UAS-GFP;* B) when compared to controls (*hs-Gal4>UAS-GFP UAS-GFP;* A) (**P* < 0.05). The graph shows means ± s.e.m.

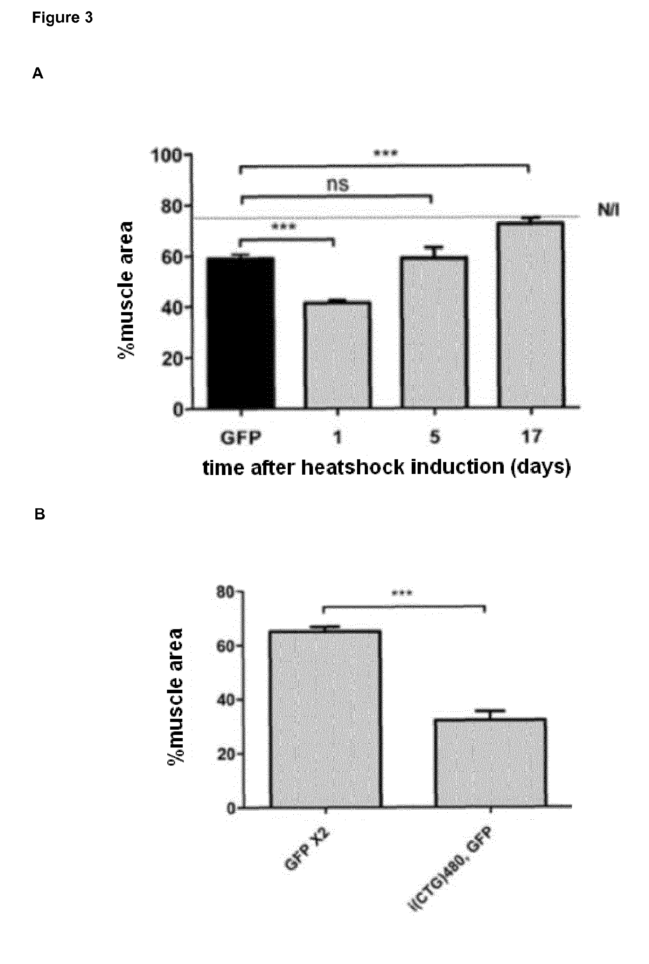

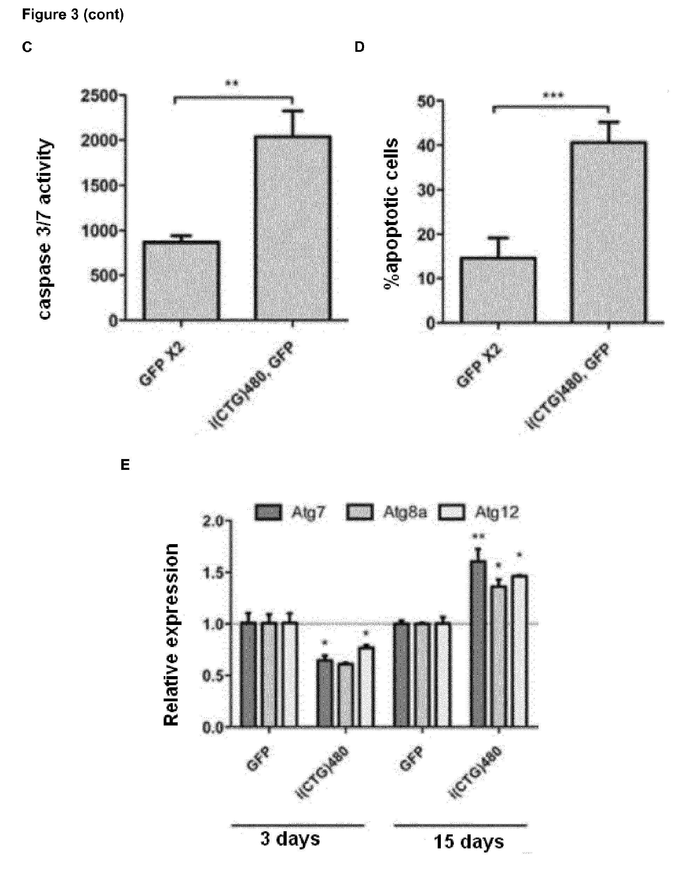

**Figure 3****.** CTG-induced muscle atrophy is reversible. The developmental expression of expanded CTG repeats increases apoptosis and autophagy.
   **(A)** Quantification of muscle mean area at different time points after HS induction *(hs-Gal4 UAS-i(CTG)480>UAS-GFP)* showed a recovery of muscle size just five days post-induction, showing the reversibility of CTG-induced toxicity. **(B)** Quantification of IFM muscle area in resin-embedded thoraces of flies carrying myosin heavy chain-driven expression of CTG in skeletal muscle (*Mhc-Gal4 UASi(CTG)480>UAS-GFP)* which originated a 50% decrease in muscle mean area of IFM when compared to that for controls (*Mhc-Gal4>UAS-GFP UAS-GFP)* in three-day-old flies. **(C)** Increased apoptosis levels characterized by a 2-fold increment in caspase 3/7 activity. **(D)** Quantification of the percentage of nuclei containing fragmented chromatin from representative fluorescent micrographs of rostrocaudal cryosections from adult *Drosophila* thoraces of control (*GFP X2*) and DM1 model flies (*i(CTG)480, GFP).* Fragmented DNA was stained with TMR and nuclei were counterstained with DAPI. A 3-fold increase in the percentage of nuclei containing fragmented chromatin was obtained in model flies when compared to that for controls. **(E)** Time-dependent increased autophagy was observed in model flies. The q-PCR of samples of 3 days after hatching showed a reduced expression of *Atg7, Atg8a* and *Atg12.* However, the same experiment performed in 15-day-old flies showed much higher levels of gene expression, while expression levels of the same genes in control flies (*Mhc-Gal4>UAS-GFP UAS-GFP)* remained unchanged. (**P* < 0.05, ***P* < 0.001, ****P* < 0.0001). Error bars are standard deviations.

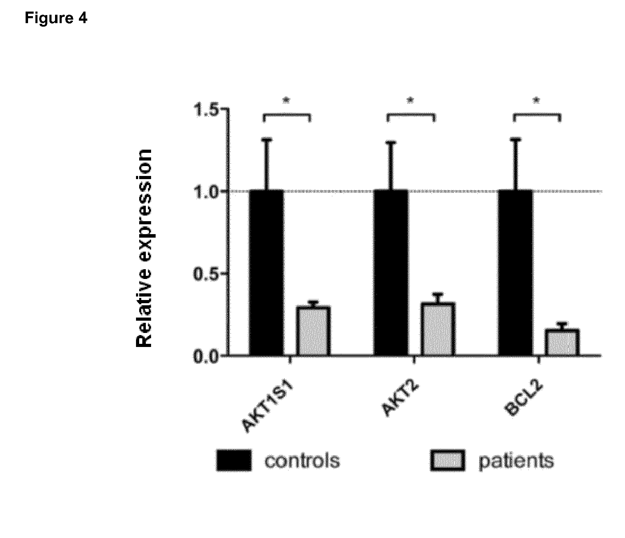

**Figure 4****.** Autophagy and apoptosis related genes are misregulated in human DM1 muscle biopsies.
   Quantification of relative expression levels of genes involved in apoptosis and autophagy regulation from mRNA obtained from human skeletal muscle biopsies from six patients and six controls. *GADPH* was used as the endogenous control. Graph bars represent average fold changes of gene expression, calculated by the 2^{-ΔΔCt} method, and their standard errors. **P* < 0.05.

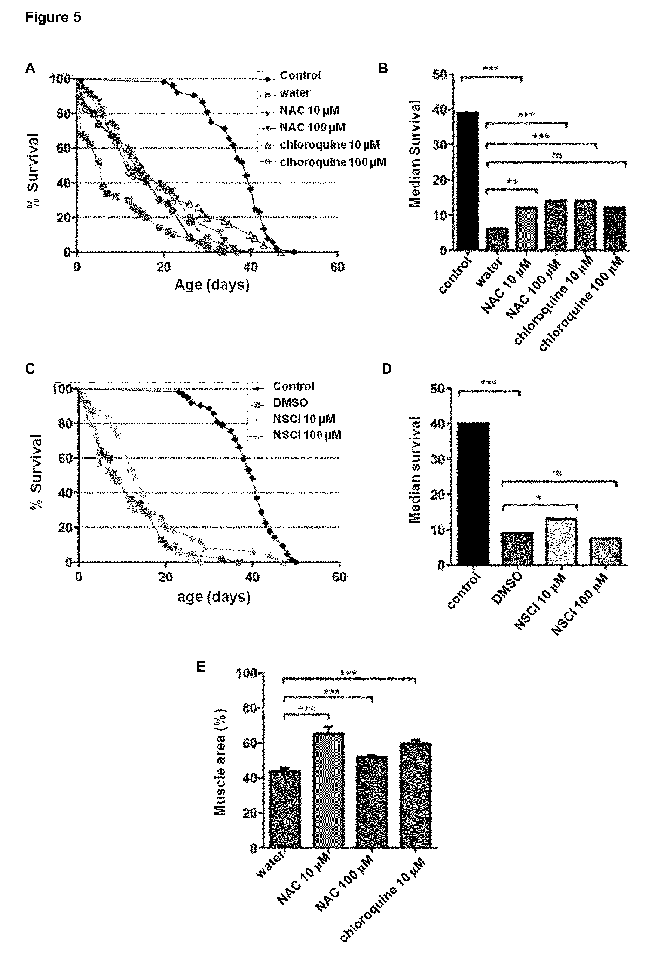

**Figure 5****.** Autophagy or apoptosis inhibition improve viability.
   **(A)** Survival curves obtained from chemical inhibition of autophagy by oral administration of 10 µM and 100 µM of NAC (● and ▼, respectively) or 10 µM and 100 µM chloroquine (△ and ◊, respectively) and **(B)** the associated representation of median survival obtained from said survival curves, whereby decreased median survival is observed in model flies as a result of CTG repeat toxicity (compare control and water bars). Said data shows an increased median survival when compared to that for untreated flies (*Mhc-Gal4 UASi(CTG)480>yw)* (water). **(C)** Survival curves obtained from chemical inhibition of apoptosis by oral administration of 10 µM and 100 µM NSCI (● and ▲) and **(D)** the associated representation of median survival obtained from said survival curves, whereby decreased median survival is observed in model flies as a result of CTG repeat toxicity (compare control and DMSO bars). Said data shows an improvement in median survival when compared to that for the untreated when lower concentration of NSCI was tested. No effect was obtained when flies were fed with food containing 100 µM NSCI (**P* < 0.05, ***P* < 0.001, ****P* < 0.0001). **(E)** Percentage muscle area increase of IFM observed in DM1 model flies after oral administration of 10 µM or 100 µM of NAC, or 10 µM of chloroquine, as opposed to water as a control (****P* < 0.0001).

### Detailed Description of the Invention

The present invention relates to a compound, or a salt thereof, which is an autophagy inhibitor and/or an apoptosis inhibitor, for use in the prevention and/or treatment of myotonic dystrophy type 1 in a patient. Thus, the present invention also relates to use of a compound, or a salt thereof, which is an autophagy inhibitor and/or an apoptosis inhibitor, in the manufacture of a medicament for the prevention and/or treatment of myotonic dystrophy type 1 in a patient.

In the present invention, the autophagy inhibitor is preferably a macroautophagy inhibitor. A macroautophagy inhibitor is a compound that inhibits the formation of an autophagosome and/or fusion of said autophagosome with a lysosome, in particular by inhibiting the formation of a double membrane around a cytoplasmic substrate and/or the delivery of said cytoplasmic substrate to a lysosome. Alternatively, in the present invention the apoptosis inhibitor is preferably a caspase 3 inhibitor. A caspase 3 inhibitor is a compound that inhibits the activation of caspase 3.

In the present invention, the aforementioned compound for use in the prevention and/or treatment of myotonic dystrophy type 1 is selected from:
a) a compound selected from the formula 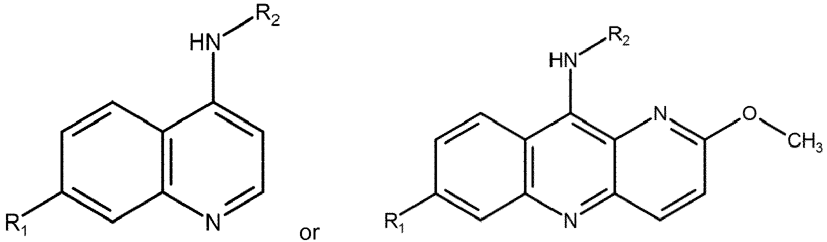 whereby
   R₁ is selected from H or a halide; and
   R₂ is selected from an alkyl moiety substituted with a moiety selected from an amino, alkyl amino, dialkyl amino, cycloamino or hydroxyalkyl amino moiety or a phenolic moiety substituted with at least one moiety selected from a aminoalkyl, (alkylamino)alkyl, (dialkylamino)alkyl, (cycloamino)alkyl or (hydroxyalkylamino)alkyl moiety;
b) a compound of the formula R³SCH₂-CH(NHR⁴)-COR⁵, wherein
   R³ is selected from H, -CH₂CH₂CH₂OH or -CH₂COOH;
   R⁴ is selected from H or -C(=O)CH₃; and
   R⁵ is selected from -OH, -OCH₃ or -NH₂; and
   whereby R³ and R⁴ cannot both be H when R⁵ is -OH; or
c) a compound of the formula 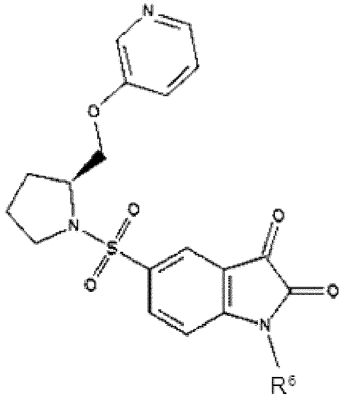 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties,
or a salt thereof.

In one aspect of this invention, the autophagy inhibitor for use in the prevention and/or treatment of myotonic dystrophy type 1 is selected from a 4-aminoquinoline compound or salts thereof, wherein said 4-aminoquinoline compound is a compound selected from the formula 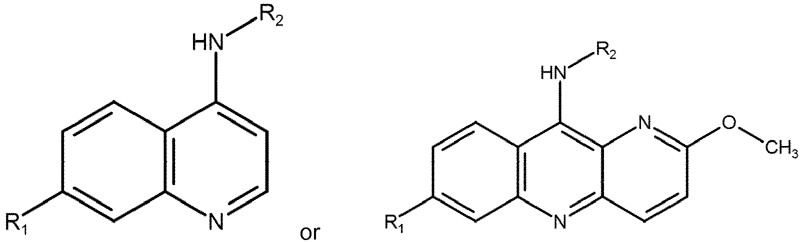 whereby
R₁ is selected from H or a halide; and
R₂ is selected from
   - an alkyl moiety substituted with a moiety selected from an amino, alkyl amino, dialkyl amino, cycloamino or hydroxyalkyl amino moiety; or
   - a phenolic moiety substituted with at least one moiety selected from a aminoalkyl, (alkylamino)alkyl, (dialkylamino)alkyl, (cycloamino)alkyl or (hydroxyalkylamino)alkyl moiety.

Preferably, the 4-aminoquinoline compound is a compound comprising a 4-amino-7-chloroquinoline moiety *(i.e.* a 4-amino-7-chloroquinoline compound) or a 7-chloro-10-amino-pyrido[3,2-b]quinoline moiety *(i.e.* a 7-chloro-10-amino-pyrido[3,2-*b*]quinoline compound), wherein R₁ is Cl. In one preferred embodiment, said compound for use in the prevention and/or treatment of myotonic dystrophy type 1 is selected from one of chloroquine, amodiaquine, hydroxychloroquine, pyronaridine or desethylchloroquine, or a salt thereof selected from phosphoric acid, sulfuric acid, hydrochloric acid, hydrobromic acid or hydroiodic acid salts thereof.

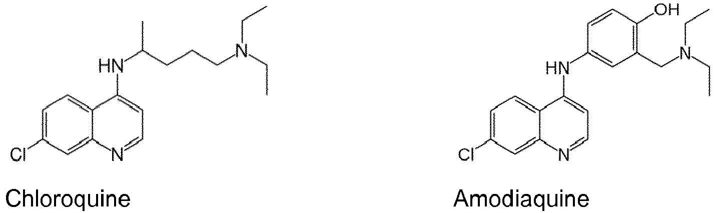

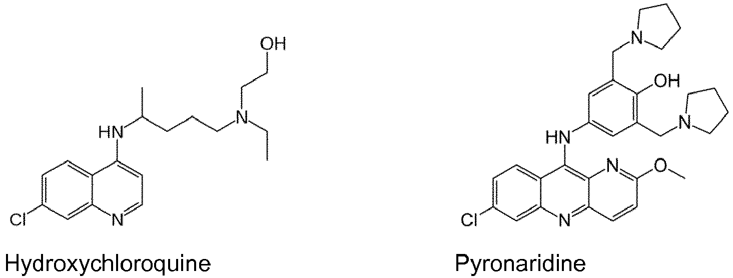

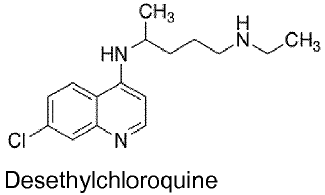

More preferably, said 4-aminoquinoline compound is selected from one of chloroquine, chloroquine phosphate, chloroquine sulfate, chloroquine hydrochloride, amodiaquine hydrochloride, hydroxychloroquine sulfate, pyronaridine phosphate or desethylchloroquine phosphate. Even more preferably, R₁ is Cl and R₂ is -CH(CH₃)CH₂CH₂CH₂NR²¹(CH₂CH₃), wherein R²¹ is selected from H, -CH₂CH₃ or -CH₂CH₂OH. Furthermore preferably, said 4-aminoquinoline compound is selected from one of chloroquine, chloroquine phosphate, chloroquine sulfate, chloroquine hydrochloride or hydroxychloroquine sulfate, most preferably chloroquine, chloroquine phosphate, chloroquine sulfate, chloroquine hydrochloride. Alternatively and furthermore preferable, said compound for use in the prevention and/or treatment of myotonic dystrophy type 1 is chloroquine or a phosphate, sulfate or chloride salt thereof. In a preferred embodiment of the invention, said chloroquine is (*R*)-*N'*-(7-chloroquinolin-4-yl)-*N,N*-diethyl-pentane-1,4-diamine and/or (*S*)-*N'*-(7-chloroquinolin-4-yl)-*N,N*-diethyl-pentane-1,4-diamine, or salts thereof. In another preferred embodiment of the present invention, said chloroquine is a racemic mixture of (*R*)-*N'*-(7-chloroquinolin-4-yl)-*N,N*-diethyl-pentane-1,4-diamine and/or (S)-*N*'-(7-chloroquinolin-4-yl)-*N,N*-diethyl-pentane-1,4-diamine.

In another aspect of this invention, the autophagy inhibitor for use in the prevention and/or treatment of myotonic dystrophy type 1 is a compound of the formula R³SCH₂-CH(NHR⁴)-COR⁵, or salts thereof, wherein
R³ is H, -CH₂CH₂CH₂OH or -CH₂COOH;
R⁴ is H or -C(=O)CH₃; and
R⁵ is -OH, -OCH₃ or -NH₂; and
whereby R³ and R⁴ cannot both be H when R⁵ is -OH. Thus, preferably, said compound for use in the prevention and/or treatment of myotonic dystrophy type 1 is selected from one of *N*-acetyl L-cysteine, *N*-acetylcysteine amide, carbocisteine, fudosteine or mecysteine, or a salt thereof.

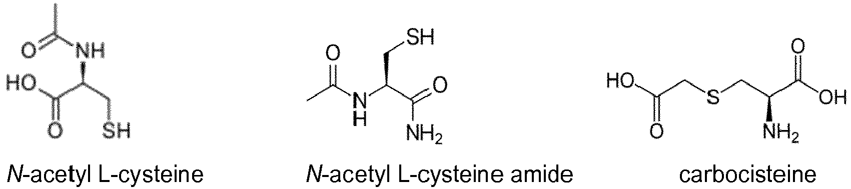

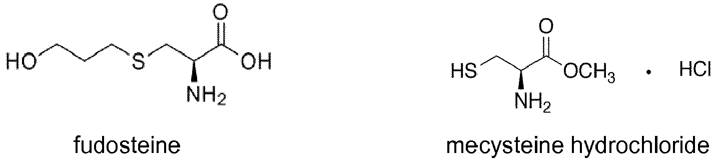

In a yet more preferred embodiment, the autophagy inhibitor for use in the prevention and/or treatment of myotonic dystrophy type 1 is a compound of the formula R³SCH₂-CH(NHR⁴)-COR⁵, or salts thereof, wherein R³ is H, R⁴ is -C(=O)CH₃ and R⁵ is -OH or-NH₂. Even more preferably, said compound for use in the prevention and/or treatment of myotonic dystrophy type 1 is selected from one of *N*-acetyl L-cysteine, *N*-acetyl L-cysteine lysine salt or *N*-acetyl L-cysteine amide. Most preferably, said compound is *N*-acetyl L-cysteine.

In an alternative aspect of this invention, the apoptosis inhibitor for use in the prevention and/or treatment of myotonic dystrophy type 1 is a compound of the formula 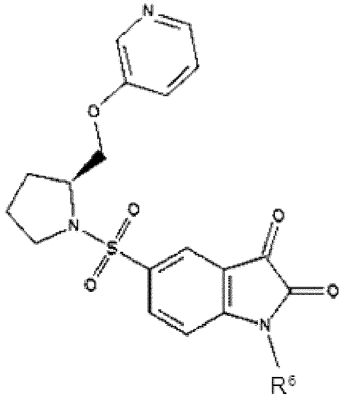 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties, or a salt thereof. Preferably, the compound for use in the prevention and/or treatment of myotonic dystrophy type 1 is selected from 1-(4-methoxybenzyl)-5-({(2*S*)-2-[(3-pyridinyloxy)methyl]-1-pyrrolidinyl}sulfonyl)-1*H*-indole-2,3-dione, 1-(4-hydroxybenzyl)-5-({(2S)-2-[(3-pyridinyloxy)methyl]-1-pyrrolidinyl}sulfonyl)-1*H*-indole-2,3-dione, 1-(4-bromobenzyl)-5-({(2*S*)-2-[(3-pyridinyloxy)methyl]-1-pyrrolidinyl}sulfonyl)-1*H*-indole-2,3-dione, or 1-[4-(2-fluoroethoxy)benzyl]-5-({(2*S*)-2-[(3-pyridinyloxy)methyl]-1-pyrrolidinyl}sulfonyl)-1*H*-indole-2,3-dione. More preferably, the compound for use in the prevention and/or treatment of myotonic dystrophy type 1 is 1-(4-methoxybenzyl)-5-({(2*S*)-2-[(3-pyridinyloxy)methyl]-1-pyrrolidinyl}sulfonyl)-1*H*-indole-2,3-dione (hereinafter NSCI), or a salt thereof.

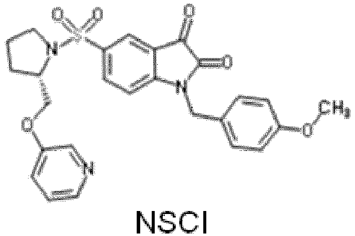

The disease or condition which may be prevented or treated in a patient by the present invention is myotonic dystrophy type 1. For the purposes of the present invention, a patient with myotonic dystrophy type 1 is a patient having more than 50 repetitions of the CTG triplet in the *DMPK* gene. However, DM1 may also be characterized by the histopathology of (i) skeletal cells, which may exhibit changes in fibre size and architecture, centronucleated fibres, ringed fibres, fibre splitting, fibrosis, sarcoplasmic masses, changes in fibre type content, type I atrophy, degenerative changes in mitochondria, sarcoplasmic reticulum, sarcotubular system and Z line; (ii) heart tissue, which may exhibit myocyte hypertrophy, interstitial fibrosis, fatty infiltration, vascular degeneration, dissolution of myofibres, degenerative changes in myofibres, sarcoplasmic reticulum, mitochondria and transverse tubular system; and/or (iii) central nervous system tissue, which may exhibit neuronal loss, neuronal inclusion bodies, presenile neurofibrillary tangles, gliosis and astrocytosis, disordered neuronal migration, enlargement of ventricules and/or progressive white-matter abnormalities. DM1 may also be characterized by its symptomatology, whereby (i) myotonia, progressive wasting and weakness, pain and/or general hypotonia (in the case of congenital DM1) may be associated with skeletal muscle; (ii) hypersomnolence, cognitive impairment, executive dysfunction, visual-spatial memory deficits, neuropsychological changes and/or (in the case of congenital DM1) mental retardation may be associated with the central nervous system; (iii) cardiac conduction defects, prolonged PR intervals, first degree atrioventricular block and/or arrhythmias may be associated with the heart; (iv) gastrointestinal complications, swallowing issues, abdominal pain, abnormal motility, malabsorption, constipation/diarrhea and/or anal incontinence may be associated with smooth muscle; and/or (v) premature subcapsular iridescent and multicoloured cataracts, hyperinsulinism (diabetes), respiratory complications, testicular atrophy, reduced fertility, male prefrontal balding and/or hypogammaglobulinemia may be associated with other organs and systems.

For the purposes of the present invention, treatment of myotonic dystrophy type 1 involves treatment of symptomatic myotonic dystrophy type 1 which restores the phenotype of a patient with said disease or condition to a non-diseased state *[i.e.* reduces the aforementioned histopathology and/or symptomatology associated with DM1, for example according to disease-specific muscular impairment rating scales (12)], whereas prevention of myotonic dystrophy type 1 involves treatment of asymptomatic myotonic dystrophy type 1 which maintains the phenotype of a patient with said disease or condition in a non-diseased state *(i.e.* prevents the onset of the aforementioned histopathology and/or symptomatology associated with DM1).

In a further preferred embodiment, the myotonic dystrophy type 1 which may be prevented or treated in a patient by the present invention is selected from severe congenital myotonic dystrophy type 1 or adult-onset myotonic dystrophy type 1. Severe congenital myotonic dystrophy type 1 is a muscular dystrophy that begins prior to adolescence and may be apparent at birth. Severe congenital myotonic dystrophy type 1 is characterized by weak muscle tone (hypotonia), an inward- and upward-turning foot (clubfoot), breathing problems, delayed development, and intellectual disability (cognitive and behavioral abnormalities). In contrast, adult-onset myotonic dystrophy type 1 is a muscular dystrophy that begins in adolescence or young adulthood. Adult-onset myotonic dystrophy type 1 begins with weakness in the muscles of the face, neck, fingers and ankles and slowly progresses to these and other muscles.

In the present invention the muscular dystrophy type 1 is a muscular dystrophy type 1 in mammals. More preferably, said muscular dystrophy type 1 is a hominine muscular dystrophy type 1, canine muscular dystrophy type 1, feline muscular dystrophy type 1, bovine muscular dystrophy type 1, ovine muscular dystrophy type 1, porcine muscular dystrophy type 1, equine muscular dystrophy type 1, camelline muscular dystrophy type 1, caprine muscular dystrophy type 1 or cervine muscular dystrophy type 1. Furthermore preferably, said muscular dystrophy type 1 is a muscular dystrophy type 1 in a human, dog, cow, horse or camel, even more preferably in a human or dog, and most preferably in a human patient.

In a particularly preferred embodiment, the present invention discloses a compound for use in the treatment of myotonic dystrophy type 1 in a patient, wherein said compound is selected from chloroquine, chloroquine phosphate, chloroquine sulfate, chloroquine hydrochloride, hydroxychloroquine sulfate, N-acetyl L-cysteine, L-cysteine lysine salt, N-acetylcysteine amide, carbocisteine, 1-(4-methoxybenzyl)-5-({(2S)-2-[(3-pyridinyloxy)methyl]-1-pyrrolidinyl}sulfonyl)-1*H*-indole-2,3-dione, 1-(4-hydroxybenzyl)-5-({(2S)-2-[(3-pyridinyloxy)methyl]-1-pyrrolidinyl}sulfonyl)-1*H*-indole-2,3-dione, or 1-(4-ethoxybenzyl)-5-({(2*S*)-2-[(3-pyridinyloxy)methyl]-1-pyrrolidinyl}sulfonyl)-1*H*-indole-2,3-dione. More preferably, said compound for use in the prevention and/or treatment of myotonic dystrophy type 1 is selected from chloroquine, chloroquine phosphate, chloroquine sulfate, chloroquine hydrochloride, *N*-acetyl L-cysteine, *N*-acetyl L-cysteine amide, 1-(4-methoxybenzyl)-5-({(2S)-2-[(3-pyridinyloxy)methyl]-1-pyrrolidinyl}sulfonyl)-1*H-*indole-2,3-dione, or 1-(4-hydroxybenzyl)-5-({(2*S*)-2-[(3-pyridinyloxy)methyl]-1-pyrrolidinyl}sulfonyl)-1*H*-indole-2,3-dione.

In an even more particularly preferred embodiment, the present invention relates to a compound, or a salt thereof, for use in the treatment of myotonic dystrophy type 1, wherein said compound is selected from:
a) chloroquine;
b) *N*-acetyl L-cysteine; or
c) NSCI.

The present invention also relates to a pharmaceutical composition for use in the treatment of myotonic dystrophy type 1 according to the present description, in a patient comprising:
I) at least one compound, or a salt thereof, selected from an autophagy inhibitor or an apoptosis inhibitor according to the present description; and
II) at least one excipient and/or carrier.

Thus, the present invention also relates to use of a pharmaceutical composition in the manufacture of a medicament for the prevention and/or treatment of myotonic dystrophy type 1 in a patient, wherein said pharmaceutical composition comprises:
I) at least one compound, or a salt thereof, selected from an autophagy inhibitor or an apoptosis inhibitor according to the present description; and
II) at least one excipient and/or carrier.

In particular, the present invention relates to a pharmaceutical composition for use in the treatment of myotonic dystrophy type 1 in a patient comprising:
I) at least one compound, or a salt thereof, selected from
   a) a compound selected from the formula 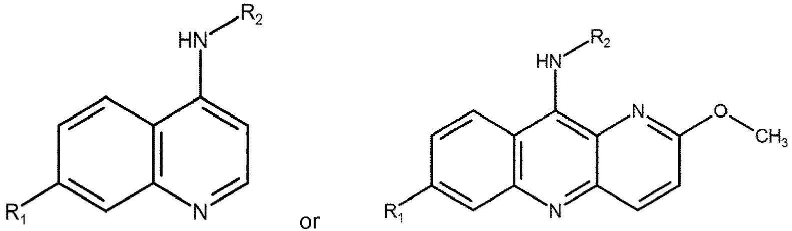 whereby
      R₁ is selected from H or a halide; and
      R₂ is selected from an alkyl moiety substituted with a moiety selected from an amino, alkyl amino, dialkyl amino, cycloamino or hydroxyalkyl amino moiety; or a phenolic moiety substituted with at least one moiety selected from a aminoalkyl, (alkylamino)alkyl, (dialkylamino)alkyl, (cycloamino)alkyl or (hydroxyalkylamino)alkyl moiety;
   b) a compound of the formula R³SCH₂-CH(NHR⁴)-COR⁵, wherein
      R³ is selected from H, -CH₂CH₂CH₂OH or -CH₂COOH;
      R⁴ is selected from H or -C(=O)CH₃; and
      R⁵ is selected from -OH, -OCH₃ or -NH₂; and
      whereby R³ and R⁴ cannot both be H when R⁵ is -OH; or
   c) a compound of the formula 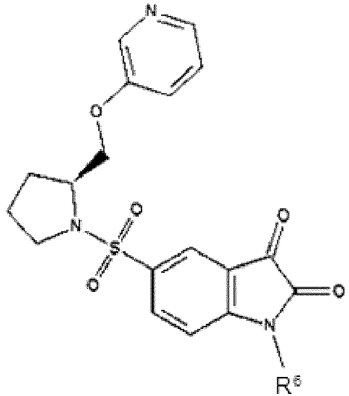 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties; and
II) at least one excipient and/or carrier.

Preferably, the pharmaceutical composition for use in the treatment of myotonic dystrophy type 1 in a patient comprises:
I) at least one compound, or a salt thereof, selected from:
   a) chloroquine;
   b) N-acetyl L-cysteine; or
   c) NSCI; and
II) at least one excipient and/or carrier.

The excipient and/or carrier comprised in said pharmaceutical composition for use in the prevention and/or treatment of myotonic dystrophy type 1 of the present invention may be an inert ingredient or ingredients. Said excipient or carrier may be a diluent, as a way of example. Such compositions can be in crystalline, powder, granular, compacted solid, liquid, solution, suspension, elixir, syrup, emulsion, cream, gel, droplet, mist, vapor or spray form. In making the compositions, conventional techniques for the preparation of pharmaceutical compositions may be used. For example, the pharmaceutical composition described herein may be comprised in a capsule, tablet, pill, caplet, ampoule, sachet, syringe, cartridge, nebulizer or other container. Thus, the present invention additionally comprises a kit of parts comprising the pharmaceutical composition of the present invention and a container, wherein said pharmaceutical composition is contained in said container. Preferably, said container is preferably a blister pack, capsule, ampoule, sachet, bottle, vial, syringe or nebulizer, more preferably, said container is a blister pack, capsule, ampoule, bottle or syringe, furthermore preferably a blister pack, ampoule or bottle, most preferably a blister pack or bottle.

The compound described herein will usually be mixed with a carrier, or diluted by a carrier, or enclosed within a carrier. When the carrier serves as a diluent, it may be solid, semi-solid, or liquid material that acts as a vehicle, excipient, or medium for the active compound. The compound of interest can be adsorbed on a granular solid carrier, for example in a sachet. Some examples of suitable carriers are solvents such as water, salt solutions such as isotonic saline, alcohols, polyethylene glycols, polyhydroxyethoxylated castor oil, peanut oil or olive oil, or other carriers such as lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose, and polyvinylpyrrolidone. Similarly, the carrier or diluent may include any sustained release material known in the art, such as glyceryl monostearate or glyceryl distearate, alone or mixed with a wax. The composition of the invention may also comprise wetting agents, emulsifying and suspending agents, preserving agents, sweetening agents or flavouring agents.

The composition for use in the prevention and/or treatment of myotonic dystrophy type 1 of the invention may be formulated so as to provide quick, sustained, or delayed release of the active ingredient after administration to the patient by employing procedures well known in the art. In addition, the pharmaceutical compositions can be sterilized and mixed, if desired, with auxiliary agents, emulsifiers, salt for influencing osmotic pressure, buffers and/or colouring substances and the like, which do not deleteriously react with the compound of the invention.

Thus, another preferred embodiment of the present invention comprises a method for producing a pharmaceutical composition according to that described herein, which comprises mixing:
I) a compound, or a salt thereof, of the formula 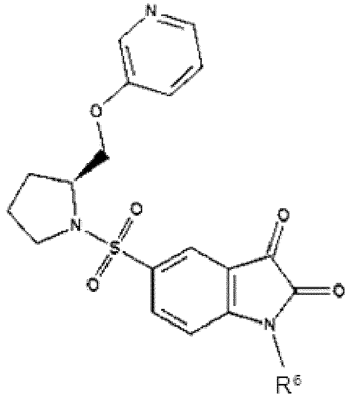 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties; and
II) at least one excipient and/or carrier.

Said method may involve a step of heating, agitation, centrifugation and/or filtration in order to ensure homogeneity of the resulting mixture.

The present invention also relates to a method of prevention and/or treatment of myotonic dystrophy type 1 in a patient, said method comprising administering to said patient at least one compound, a salt thereof or a pharmaceutical composition comprising the same in a pharmaceutically effective dose, according to a continuous schedule having a dosing interval selected from the group consisting of continuous dosing, once-daily dosing, twice-daily dosing, thrice-daily dosing, four-times daily dosing, five-times daily dosing, six-times daily dosing, once-weekly dosing, twice-weekly dosing, thrice-weekly dosing, four-times weekly dosing, five-times weekly dosing, six-times weekly dosing, biweekly dosing and twice-monthly dosing, wherein said compound is selected from
a) a compound selected from the formula 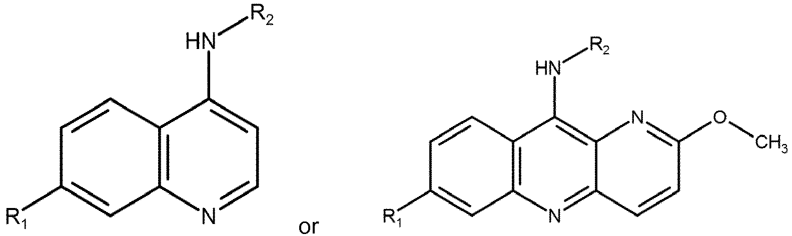 whereby
   R₁ is selected from H or a halide; and
   R₂ is selected from an alkyl moiety substituted with a moiety selected from an amino, alkyl amino, dialkyl amino, cycloamino or hydroxyalkyl amino moiety; or a phenolic moiety substituted with at least one moiety selected from a aminoalkyl, (alkylamino)alkyl, (dialkylamino)alkyl, (cycloamino)alkyl or (hydroxyalkylamino)alkyl moiety;
b) a compound of the formula R³SCH₂-CH(NHR⁴)-COR⁵, wherein
   R³ is selected from H, -CH₂CH₂CH₂OH or -CH₂COOH;
   R⁴ is selected from H or -C(=O)CH₃; and
   R⁵ is selected from -OH, -OCH₃ or -NH₂; and
   whereby R³ and R⁴ cannot both be H when R⁵ is -OH; or
c) a compound of the formula 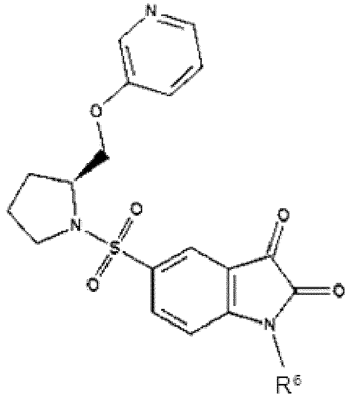 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties.

Preferably said dosing interval is selected from the group consisting of continuous dosing, once-daily dosing, twice-daily dosing, thrice-daily dosing, once-weekly dosing, twice-weekly dosing and thrice-weekly dosing and said pharmaceutically effective dose is an amount of between 0.01 to 500 mg/kg per administration. More preferably, said dosing interval is selected from the group consisting of continuous dosing, once-daily dosing, twice-daily dosing, thrice-daily dosing, four-times-daily dosing, five-times-daily dosing or six-times-daily dosing, in an amount of between 5 to 140 mg/kg per administration from the time of first detecting the myotonic dystrophy type 1. In a furthermore preferable embodiment, N-acetyl cysteine is administered in an initial dose of 140 mg/kg and subsequent doses of 70 mg/kg every 4 hours thereafter, preferably until the phenotype of a patient with said disease or condition is restored to a non-diseased state. In an analogous furthermore preferable embodiment, chloroquine is administered in an initial dose of 10 mg/kg (up to a maximum of 600 mg) and subsequent doses of 5 mg/kg (up to a maximum of 300 mg) every 6 hours, preferably until the phenotype of a patient with said disease or condition is restored to a non-diseased state. In any of the aforementioned methods, administration of the pharmaceutically effective dose can be carried out at any of the aforementioned dosing intervals by single administration in the form of an individual dose unit or by multiple administration in several smaller dose units. Alternatively, the dose may be provided as a continuous infusion over a prolonged period. Dose ranges of the pharmaceutical compositions can be adjusted as necessary for the treatment of individual patients and according to the specific condition treated

Moreover, the compound or composition for use in the prevention and/or treatment of myotonic dystrophy type 1 of the present invention may be administered to the human body by any common administration route, including invasive and non-invasive methods. In particular, administration may be performed orally, ocularly, nasally, pulmonarally, hypodermically, hyperdermically (topically), intravenously, sublingually, buccally, rectally or intravaginally. Preferably, said administration is oral, nasal, hypodermic, rectal or intravaginal, more preferably oral or rectal, most preferably oral.

In any of the aforementioned methods, administration is carried out so as to achieve an improvement in at least one symptom associated with myotonic dystrophy type 1 in a patient, either in whole or in part. Preferably, the at least one or more symptom associated with myotonic dystrophy type 1 is improved by at least 10%, more preferably by at least 20%, yet more preferably by at least 40%, furthermore preferably by at least 60%, even more preferably by 90%, or most preferably by 100% relative to the level of said at least one symptom prior to administration.

The Examples of the compounds and compositions for use in the prevention and/or treatment of myotonic dystrophy type 1 of the present invention and representative processes for their manufacture which appear below, should not be construed to limit the invention.

### Examples

### Materials and Methods

### a) Non-fluorescent histological analysis

Analysis of the indirect flight muscle area in *Drosophila* thoraces was performed as previously described (13). Briefly, six Drosophila thoraces of three-day-old females were embedded in Epon following standard procedures. After the drying of the resin, semithin sections of 1.5 µm were obtained using Ultracut E system (Reichert & Jung). Images were taken at 100x magnification with a Leica DM2500 microscope. To quantify muscle area, five images per fly containing IFMs were converted into binary ones. Considering the complete image as 100% of the area, the percentage occupied by pixels corresponding to IFMs was calculated using NIH ImageJ software. P-values were obtained using a two-tailed, non-paired t-test (α = 0.05), applying Welch's correction when necessary.

### b) qRT-PCR from fly samples

Total RNA from ten adult male flies was isolated using Tri Reagent (Sigma Aldrich). Then, 1 µg of total RNA was digested with DNase I (Life Technologies) and reverse transcribed (RT) using hexanucleotide mix (Roche Applied Science) and SuperScript II reverse transcriptase (Life Technologies). RNaseOUT (Life Technologies) was used to avoid RNA degradation. The RT reaction was performed following instructions from the provider in a GeneAmp PCR System 9700 (Applied Biosystems) thermal cycler. The qPCR was carried out using 4 ng of cDNA template for all genes, except for the reference gene, *Rp49,* where 0.4 ng were necessary. Thermal cycling was performed with SYBR Green PCR Master Mix (Applied Biosystem) following manufacturer's instructions in a StepOne Real Time thermal cycler. For primer sequences and annealing temperatures see **Table 1.** All experiments were performed using three biological replicates with three technical replicates of each. Expression levels were normalized relative to the reference gene *(Rp49)* using the 2^{-ΔΔCt} method. P-values were obtained using a two-tailed, non-paired t-test (α = 0.05), applying Welch's correction when necessary.

**Table 1. Sequence and melting temperature (Tm) of PCR primers used for standard and quantitative PCR.**

| **Primer pair** | **Forward (5 → 3')** | **SEQ. ID. NO** | **Tm (°C)** | **Reverse (5' → 3')** | **SEQ. ID.NO** | **Tm (°C)** |
|---|---|---|---|---|---|---|
| UAS | GGAAAGTCCTTGGGGTCTTC | 1 | 54 | GGAACTGATGAATGGGAGCA | 2 | 52 |
| GAL4 | CACCGACGCTAATGATGTTG | 3 | 52 | TTTGTTTTCTGCCTCCACTG | 4 | 54 |
| Atg7 | CATAGCCTGTTCAGCGGCCGT | 5 | 71 | CCGCTTGAATTCGGAGATTCCCGTC | 6 | 70 |
| Atg8a | ATCCAGACCGTGTGCCCGTCAT | 7 | 74 | ACCGACGGTCAGGTCGGAAGG | 8 | 74 |
| Atg12 | TCGATGCCAGCGAGCAAATTTTCCI | 9 | 70 | GCCCCACGCCTGATTCTTGCA | 10 | 72 |
| Rp49 | ATGACCATCCGCCCAGCATAC | 11 | 55 | ATGTGGCGGGTGCGCTTGTTC | 12 | 55 |
| AKT1S1 | AGCCCACAGAGACAGAGACC | 13 | 58 | CGTCCTCATCCATCACAAAG | 14 | 58 |
| AKT2 | CTCACACAGTCACCGAGAGC | 15 | 58 | TGGGTCTGGAAGGCATACTT | 16 | 58 |
| ATG9A | TTTGCTCAGATGGATGTTCG | 17 | 58 | TCCTCAGCTTGCTGGTACACT | 18 | 59 |
| BCL2 | CACCTGTGGTCCACCTGAC | 19 | 58 | CTGGACATCTCGGCGAAG | 20 | 59 |
| BIRC7 | CCGGTCAAAAGGAAGAGACTT | 21 | 58 | TGCGTCTTCCGGTTCTTC | 22 | 58 |
| GAPDH | AGCCACATCGCTCAGACAC | 23 | 58 | CGCCCAATACGACCAAAT | 24 | 58 |
| LAMP2 | AATGGCACAGTGAGCACAAA | 25 | 59 | GAGATGGCACAGTGGTGTGT | 26 | 58 |
| mTOR | TGCTGGAAGCCTTTGTCTATG | 27 | 59 | CGCTTGTTGCCTTTGGTATT | 28 | 59 |
| NKX3-2 | GGTGGGGTTTTCCCTGAG | 29 | 59 | GAAATTCTGAGGATTCAGGCTATG | 30 | 59 |
| VPS52 | CGGCTCCGGGTCAAGG | 31 | 62 | CTTCTGGAGGATAAACTCTCGGAT | 32 | 60 |
| VPS37 | CAGAAGGCAAAGCTGGAGA | 33 | 58 | TCCACCTGCAGAAGGTCTAAC | 34 | 59 |

### c) In situ cell death detection and hybridization

Fly thoraces of three-day-old female flies were dissected and fixed in paraformaldehyde (PFA) 4% in PBS at 4 °C overnight. The PFA was removed, and tissue was incubated in sucrose 30% for two days at 4 °C. Then, thoraces were embedded in OCT and longitudinal cryosections of 10 µm were obtained using aLeica CM 1510S cryostat. Detection of apoptotic cells in muscle tissue was performed using *In Situ* Cell Death Detection Kit (Roche Applied Science) following the manufacturer's specifications. After reaction, samples were mounted in Vectashield (Vector) with 2 µg/mL DAPI. To quantify the percentage of cells undergoing apoptosis, blue and red channels were merged and the number of nuclei containing red signal was divided by the number of total nuclei. Six representative images were analyzed per genotype. Statistical analyses were performed using a two-tailed, non-paired *t*-test (α = 0.05), applying Welch's correction when variances were significantly different. To detect CUG-RNA foci, cryosections of thoraces were washed 3 times with PBS and incubated 10 min with freshly prepared acetylation solution (DEPC water 50 ml, triethanolamine 580 µl, acetic anhydride 125 µL) and pre-hybridized for 30 min with hybridization buffer (1 µL/mL deionized formamide, 3 mM NaCl, 10 mM Tris-Cl pH 8, 0.5 mM EDTA pH 8, 100 mg/mL dextran sulfate, Denhardt's solution 1X, 0.5 mg/mL herring sperm DNA). Next, hybridization buffer and probe (SEQ ID NO: 35 1:100 Cy3-CAGCAGCAGCAGCAGCAGCA-Cy3, Sigma Aldrich) were mixed and heated at 65 °C for 5 min and cooled on ice. The sample was covered with 90 µL of the mixture with a coverslip and incubated overnight at 42 °C in a hybridization chamber. The following day samples were washed twice in SSC 20X and twice in SSC 0.5X for 15 min at 42 °C, then mounted in Vectashield (Vector) with 2 µg/mL DAPI. All images were taken at 400X magnification using a Leica DMI2500 microscope.

### d) Caspase activity determination

To detect activity levels of caspase 3/7 ten female flies of the desired genotype were homogenized in 100 µL of cold PBS buffer. After a 10 min centrifugation, supernatant was transferred into a white 96-well plate. Caspase 3/7 activity was measured using Caspase-Glo 3/7 Assay Systems (Promega). Briefly, 100 µL of Caspase-Glo 3/7 Reagent was automatically added to each well. Plates were incubated at room temperature for 30 min, and then luminescence was measured. Luminescence readings used an EnVision plate reader (PerkinElmer). Luciferase levels were normalized to total protein concentration of each sample. Total protein was measured using a BCA Protein Assay Reagent Kit (Pierce). All graphs show the average of three biological samples with three technical replicates of each. P-values were obtained using a two-tailed, non-paired *t*-test (α = 0.05).

### e) Fly strains and crosses

The *yw, UAS-GFP, UAS-2xeGFP, UAS-DIAP* and *UAS-mTOR* strains were obtained from the Bloomington *Drosophila* Stock Center (Indiana University). The *UAS-mbIC* flies have been previously described (13). So have the *UAS-IR-mbl* and *Mhc-Gal4 UAS-i(CTG)480* flies (13), as well as the *Mhc-Gal4* and *UAS-i(CTG)480* flies (15). The *hs-Gal4* flies were a gift from Dr. Galindo (Genetics and Molecular Medicine Unit, Instituto de Biomedicina de Valencia, Spain). Recombinant line *hs-Gal4 UAS-i(CTG)480* was generated during this study through genetic crossing. The presence of both transgenes in recombinant flies was confirmed by multiplexing PCR with specific primers for UAS and Gal4 transgenes. To express transgenes under the control of the *heat shock* (HS) promoter, one-day old adult offspring were placed in plastic vials and heat shocked for 1 h at 37 °C during three days. All crosses were carried out at 25 °C with standard food.

### f) Drosophila lifespan analyses

Between 50-80 newly emerged flies were collected in freshly prepared tubes containing standard nutritive medium containing apoptosis or autophagic inhibitor drugs or solvent only (water or DMSO). Males and females were kept in different tubes at 29 °C. Every day, the number of dead flies was scored. Flies were transferred to new tubes twice a week. NAC, chloroquine and NSCI (Sigma-Aldrich) were tested at 10 and 100 µM and dissolved in water or DMSO following the manufacturer's indications. Survival curves were obtained using the Kaplan-Meier method. Statistical curve comparisons were carried out according to the Gehan-Breslow-Wilcoxon test (α = 0.05).

### g) DM1 patients and skeletal muscle biopsies

Skeletal muscle biopsies used for qRT-PCR experiments were obtained in the University Hospital La Fe (Valencia, Spain) following standard protocols approved by an ethics committee. All donors gave their informed consent before extraction of samples.

### h) qRT-PCR from human samples

Human muscle biopsies were incubated overnight in 1% SDS and then homogenized in a Tissuelyser II (Qiagen) using TRIzol (Life Technologies). To perform the RT reaction, a Super Script III First-Strand Synthesis SuperMix for qRT-PCR (Life Technologies) kit was used following manufacturer's indications. In all cases 1 µg of total RNA was used as template. To carry out the qPCR, two different technologies were used according to the genes analyzed. All genes used 5 ng of cDNA as their template, and there was a final concentration of 0.25 µM of forward and reverse primers in a volume of 10 µL. Thermal cycling was performed with Fast Sybr Green Master Mix (Applied Biosystems). For primer sequences and annealing temperatures, see **Table 1.** Two genes were analyzed using TaqMan technology because of the co-amplification of unspecific products. Therefore, 5 ng of cDNA were used as a template combined with 0.5 µM of each primer, 0.1 µL of TaqMan probe (assays #81 and #44, codes 04689046001 and 04688040001, respectively, from the "Universal Probe Library", Roche Applied Science), and 5 µL of TaqMan Fast Universal PCR Master Mix (Roche Applied Science) in a total volume of 10 µL. Thermal cycling was performed following the manufacturer's instructions in a 7900HT Fast Real-Time PCR System (Applied Biosystems). Non-specific amplification corresponding to gDNA was measured using the ValidPrime method (14) and used to analyze expression levels of the genes studied. All experiments were performed using three biological replicates. Expression levels were normalized relative to the reference gene *GADPH* using the 2^{-ΔΔCt} method and GenEx (version 5.3.7) software. Pairs of samples were compared using a two-tailed *t*-test, applying Welch's correction when variances were statistically different.

### Example 1: Characterization of an inducible fly model expressing a CTG repeat expansion.

To study the role of CTG repeat expression in adult muscle mass impairment, the first inducible *Drosophila* model of DM1 was developed to study the molecular processes leading to muscle atrophy in adult muscle tissue. In particular, a DM1 fly model that expresses CTG repeats under the control of the heat-shock (HS) inducible promoter was generated, thus exhibiting muscle shrinkage as a consequence of CTG repeat expression induction.

In 1-2-day-old flies, 480 interrupted CTG repeats [i(CTG)480] under the control of the inducible heat shock (HS) promoter were induced for 1 h every day during three consecutive days (*hs-GAL4>UAS-i(CTG)480*). The present model eliminates any contribution of CTG toxicity during development and facilitates the study of adult muscle atrophy. The study of muscle morphology in dorsoventral sections of resin-embedded thoraces of control flies *(yw)* showed a significant reduction of the muscle cross-sectional area of the indirect flight muscles (IFM) (an approximately 20% reduction) comparing uninduced (N/I) and HS control flies **(****Figure 1 A)****.** Hence, this suggested that the HS treatment by itself had a deleterious impact on muscle cross-sectional area. Overexpression of the gratuitous reporter GFP under the HS promoter achieved a slight reduction of 7% in comparison to control heat-shocked flies **(****Figure 1 A)****.** Simultaneous overexpression of GFP and i(CTG)480 driven by the HS promoter displayed a significant reduction of mean muscle area after the HS (first day after third HS; **Figure 1 A)****.** There was a reduction of approximately 35% in comparison to that for counterpart flies that had not undergone heat shock **(****Figure 1 A)****.** A decrease in mean muscle area achieved by expanded CTG expression was significantly higher than the reduction caused by GFP expression (compare **Figure 1 A)****.** In fact, the muscle atrophy resulting from CTG repeat induction produced up-held wings and flightless flies, as previously described in other fly models with muscle impairment (15). Thus, overexpression of 480 CTG repeats in adult muscle results in a significant loss of muscle tissue measured in the indirect flight muscles (IFMs) of fly thoraces.

To confirm the induction of transgenes in the fly model *(hs-Gal4 UAS-i(CTG)480>UAS-*GFP), GFP expression was observed under a fluorescence dissecting microscope. Although heat shock (HS) highly induced GFP expression in flies **(****Figure 1 B)****,** a weak GFP expression was also present before the induction **(****Figure 1 C)****.** *In situ* hybridization to detect foci was carried out in adult IFM with or without HS treatment. Images showed the absence of those aggregates in both control flies **(****Figure 1 D)****,** as well as in uninduced flies **(****Figure 1 E)****.** Nevertheless, based on representative fluorescent *in situ* detection of *i(CTG)480* transcripts in longitudinal cryosections of IFM of *yw* or *hs-Gal4>UAS-i(CTG)480* uninduced or induced flies there were a large number of nuclei containing ribonuclear aggregates detected only in model flies after heat-shock treatment **(****Figure 1 F****),** a typical feature of DM1 (data not included). These data confirmed that expanded CTG repeat-induced toxicity was only triggered in adult flies after HS.

The results with the inducible model of DM1 support a role for CTG repeat expansions in adult muscle atrophy without any developmental contribution to muscle atrophy.

### Example 2: Apoptosis and autophagy are up-regulated in DM1 model flies.

Apoptosis levels and autophagy-related gene expression in the HS inducible model flies were analyzed. The number of cells undergoing apoptosis in adult muscle tissue was tested with Terminal deoxynucleotidyl transferase dUTP nick end labeling (TUNEL) **(****Figure 2****).** These data showed that model flies expressing CTG expansions *(hs-Gal4 UAS-i(CTG)480>UAS-GFP)* had approximately 30% more cells undergoing DNA fragmentation than did control flies expressing the gratuitous reporter GFP *(hs-Gal4 UAS-GFP>UAS-GFP).* In addition, caspase 3/7 activity was analyzed and found an increase of around 90% in model flies (data not included). Moreover, quantitative analyses of the expression levels of the autophagy-related genes *Atg7, Atg8a* and *Atg12* showed that autophagy was also significantly up-regulated in model flies expressing expanded CTG repeats. An increase of at least two fold was detected in comparison to that for control flies (data not included; *hs-Gal4 UAS-i(CTG)480>UAS-GFP* compared to *hs-Gal4 UAS-GFP>UAS-GFP).* Thus, muscle size reduction was concomitant with upregulation of autophagy-related genes *Atg7, Atg8a* and *Atg12,* and pathological caspase activity activation, thereby suggesting that both catalytic pathways might be involved in the muscle atrophy phenotype observed. All together, these data indicate that apoptosis and autophagy are increased in model flies.

Moreover, genetic inhibition of apoptosis or autophagy pathways provides evidence that activation of autophagy and apoptosis is responsible for CTG-induced muscle atrophy and degeneration in DM1 and identifies autophagy inhibition in particular as a potential mechanism to ameliorate muscle atrophy in adult muscle tissue.

### Example 3: Autophagy and apoptosis impairment in a myosin heavy chain-driven fly model of DM1.

The evolution over the time of the area of IFM in flies expressing expanded CTG repeat was also analyzed. The expression of RNA containing CUG repeat expansion was induced one hour a day, during three days and then the IFM area was studied. In our experimental paradigm, HS induced CTG repeat expression transitorily. The study of muscle area at different time points (1, 5 and 17 days) after HS showed that the atrophic phenotype generated by CTG repeat expression reverted with time. Surprisingly, the data suggested model reversibility, given that 5 days after HS induction, there was no difference between heat-shocked flies carrying CTG repeat expansion and uninduced model flies *(i.e.* the IFM area was almost the same), showing a recovery higher than 40% **(****Figure 3 A)****.** At 17 days after HS induction, the quantified area of IFM showed a striking increase of almost 80% in comparison to muscles analyzed 1 day after HS and, in the latter case, there was no statistically significant difference with non-induced control flies. It is worthy to mention that after 17 days, the deleterious effect of HS on muscle area was also reversed.. These data confirmed that the CTG toxic effect in muscle is reversible in adults.

To test whether autophagy and apoptosis regulation could rescue the degenerative process of CTG-expressing muscles over time, a myosin heavy chain-driven DM1 model was used. In this case, a continuous expression of CTG expansion was required to observe degenerative phenotypes. Previous studies from our laboratory showed that developmental expression of expanded CTG repeat in *Drosophila* reduced lifespan and caused muscle degeneration (15). Briefly, the effect of expressing CUG repeat RNA in *Drosophila* muscles or ubiquitously in the fly was studied with *Myosin heavy chain (Mhc)-Gal4* and *daughterless (da)-Gal4* lines, respectively, and resulted in a decrease of average survival of flies expressing i(CTG)480 in both cases. Continued expression of CTG repeat was therefore detrimental to fly survival.

In order to study a functional phenotype, the effects of apoptosis and autophagy on fly survival were analyzed. Hence, a model where expanded CTG repeats were expressed continuously was used. To formulate a deeper characterization of DM1 model flies that continuously expressed CUG repeat RNA over the time, the same parameters studied in the inducible model using the *Mhc-Gal4* driver in three-day-old flies were analyzed. *Mhc-Gal4>UAS-i(CTG)480* flies also showed an atrophic phenotype in IFM muscles resulting in a 50% decrease in muscle area in comparison with that for GFP expressing flies **(****Figure 3 B)****.** Muscle atrophy was more acute in this model fly than in the inducible one. To determine apoptosis levels the activity of caspase 3/7 and the percentage of apoptotic cells with TUNEL were studied. The expression of CUG containing RNA resulted in an increase of apoptosis, achieving a 2.2-fold increase of caspase 3/7 activity **(****Figure 3 C)****.** Concomitant to these data, TUNEL assays showed a 2.7-fold increase of cells undergoing apoptosis in flies carrying RNA containing CTG repeat **(****Figure 3 D)****.** Expression levels of *Atg7, Atg8a* and *Atg12* were downregulated in DM1 model flies. However, in 15-day-old flies, an increased expression of autophagy-related genes was detected, which was not detected in age-matched counterpart flies. Comparing flies with ages of 3 and 15 days, a significant 2.5-fold increase of *Atg7, Atg8a* and *Atg12* expression was quantified only in flies expressing CTG repeat, while in control flies, the expression of these genes was not altered over the time **(****Figure 3 E)****.**

Thus, the data described herein showed phenotypes similar to those observed in the inducible model: increased muscle atrophy, apoptosis and autophagy. Differences in muscle mean area between model flies and controls, however, were greater than those observed in the inducible model. This suggests not only that the expression of RNA containing CUG expansion for longer periods of time has more deleterious effects on flies, but also that expression levels could be different depending on the promoter used. In this model, a correlation between a shortening of lifespan and an increased autophagy-related gene expression over time was found.

### Example 4: Expression of apoptosis and autophagy related genes is altered in DM1 patients.

In order to validate the relevance of the findings in *Drosophila* DM1 models, qRT-PCR was used to study the expression levels of ten genes related to apoptosis and/or autophagy in skeletal muscle biopsies from six DM1 patients (aged 33 ± 4) compared with those of the biopsies from six healthy individuals (aged 39 ± 4) and normalized against endogenous *GADPH.* Detailed information of the samples is contained in **Table 2.** The genes analyzed were *AKT1S1*, *AKT2, ATG9A, BCL2, BIRC7, LAMP2, mTOR, NKX3-2, VPS52* and VPS37. Statistical analyses revealed a significant reduction in the levels of *AKT1S1* (78%; P = 0.044), *AKT2* (81%; P = 0.035) and *BCL2* (90%; P = 0.043) **(****Figure 4****).** The confirmed genes encoded proteins that negatively regulate apoptosis and/or autophagy. AKT1 S1, also known as Pras40, is an anti-autophagic protein present in mTOR Complex 1 (mTORC1) [reviewed in (16)], a key component in the regulation pathway of autophagy. AKT1, AKT2 and AKT3 form the AKT family. AKT inhibition triggers the induction of apoptosis and autophagy (17). Moreover, the silencing of AKT2 resulted in an increase of LCII, which means that its inhibition contributes to autophagy induction (18). Finally, BCL2 encodes an integral outer mitochondrial membrane protein that blocks apoptotic death. Together these results confirm that both catalytic pathways which were found impaired in model flies were also altered in patients.

**Table 2. Sex, age and CGT expansion size (kb) in peripheral blood of DM1 patients and healthy controls**

| **DM1 Patients** | | | **Controls** | |
|---|---|---|---|---|
| **Gender** | **Age** | **CTG expansion (kb)** | **Gender** | **Age** |
| Male | 51 | 3 | Male | 58 |
| Male | 29 | 2 | Male | 48 |
| Male | 28 | 0.3 | Male | 31 |
| Male | 27 | 1.1 | Male | 31 |
| Male | 27 | 0.75 | Male | 24 |
| Male | 20 | 1 | Male | 25 |

Thus, the over-activation of apoptosis and autophagy pathways observed in model flies was confirmed in human samples by downregulation in skeletal muscle biopsies of genes that inhibit one or both pathways, *AKT2, AKT1S1* and *BCL2.* Furthermore, in a previously published study using the same human samples as in the present work, there was an overexpression of *ATG4,* a gene involved in autophagosome formation (19).

### Example 5: Chemical inhibition of apoptosis or autophagy rescues DM1 lifespan.

The effect of a chemical inhibition of apoptosis or autophagy in model flies (*Mhc-Gal4 UASi(CTG)480>yw)* was studied. Consequently, two different drugs previously described as autophagic inhibitors were tested. N-acetyl cysteine (NAC) blocks reactive oxygen species (ROS) and protects cells by inhibiting autophagy through activation of the PI3K/Akt/mTOR pathway (20), and chloroquine triggers autophagy inhibition raising lysosomal pH and, consequently, the fusion of autophagosome with lysosome, thus preventing lysosomal protein degradation [reviewed in (21)]. Both drugs were assayed at two different concentrations, 10 and 100 µM (**Figure 5** **A, B**). In all cases, an increase in mean life of at least twice compared to that for untreated flies was observed. Moreover, it was additionally observed that DM1 model flies similarly administered with 10 µM and 100 µM of NAC and 10 µM of chloroquine resulted in an increase in muscle area as opposed to those flies treated with water as a control **(****Figure 5 E)****.**

Additionally, the effects of apoptosis inhibition were assayed. NSCI, a caspase 3 inhibitor, was orally administered. Like for the autophagy inhibitors, tests were performed at 10 and 100 µM and an improvement of mean life of 4 days was observed at the lower concentration (**Figure 5** **C, D**).

These results reveal that chemical inhibition of autophagy and/or apoptosis was a valid strategy for improving survival and restore muscle mass in model DM1 flies. In particular, the two tested autophagy inhibitor drugs, NAC and chloroquine (which are presently available for the treatment of other conditions), more than doubled the survival in myotonic dystrophy type 1 model flies. In contrast, WO 2012/164234 A1 reports a much lower improvement in survival of from 22 to 37 days in model mice (9). Moreover, chemical inhibition of apoptosis by oral administration of NSCI in adult flies also improved the mean life expectancy of myotonic dystrophy type 1 model flies.

The success of the rescue experiments and the reversibility of the atrophic phenotype observed in the inducible model generated in this study provide proof of principle for therapeutic strategies aimed at limiting autophagy in adult muscles.

It is significant that chemical inhibition of apoptosis by oral administration of NSCI in adult flies had beneficial effects in fly mean life. These results are interesting because although apoptosis inhibition throughout the development was pernicious for fly survival, its inhibition during adulthood was able to improve the mean life of model flies. Thus, increased apoptosis also contributes to the toxic effects caused by expanded CTG expression.

Chloroquine achieves autophagy inhibition by disrupting autolysosome formation. So increased autophagosome formation shown by the overexpression of *ATG4* was offset by chloroquine action. Moreover, an alteration of the AKT pathway in DM1 is consistent with NAC being able to improve survival in flies, since this chemical compound inhibits autophagy through activation of the PI3K/Akt/mTOR pathway (20). It is noteworthy that two tested autophagy inhibitor drugs, NAC and chloroquine, are presently available for the treatment of other conditions.

In conclusion, these findings demonstrate the existence of a defect in apoptosis and autophagy in two different fly models and in humans, and that a treatment with drugs to restore either of both or these pathways enhances survival in flies. The inhibition of muscle atrophy observed in adult muscle tissue is a novel candidate for improving DM1 therapy.

### References

1. Harper, P. (2001) 3rd ed., Myotonic dystrophy. Saunders, London.
2. Gagnon, C., Noreau, L., Moxley, R.T., Laberge, L., Jean, S., Richer, L., Perron, M., Veillette, S. and Mathieu, J. (2007) Towards an integrative approach to the management of myotonic dystrophy type 1. J. Neurol. Neurosurg. Psychiatry, 78, 800 - 806.
3. Sicot, G., Gomes-Pereira M. (2013) RNA toxicity in human disease and animal models: From the uncovering of a new mechanism to the development of promising therapies. Biochim. Biophys. Acta, 1832,1390 - 1409
4. Peter, A.K. and Crosbie, R.H. (2006) Hypertrophic response of Duchenne and limb-girdle muscular dystrophies is associated with activation of Akt pathway. Exp. Cell Res., 312, 2580 - 2591.
5. De Palma, C., Morisi, F., Cheli, S., Pambianco, S., Cappello, V., Vezzoli, M., Rovere-Querini, P., Moggio, M., Ripolone, M., Francolini, M. et al. (2012) Autophagy as a new therapeutic target in Duchenne muscular dystrophy. Cell Death Dis., 3, e418.
6. Grumati, P., Coletto, L., Sabatelli, P., Cescon, M., Angelin, A., Bertaggia, E., Blaauw, B., Urciuolo, A., Tiepolo, T., Merlini, L. et al. (2010) Autophagy is defective in collagen VI muscular dystrophies, and its reactivation rescues myofiber degeneration. Nat. Med., 16, 1313 - 1320.
7. Grumati, P., Coletto, L., Sandri, M. and Bonaldo, P. (2011) Autophagy induction rescues muscular dystrophy. Autophagy, 7, 426-428.
8. Sandri, M., Coletto, L., Grumati, P. and Bonaldo, P. (2013) Misregulation of autophagy and protein degradation systems in myopathies and muscular dystrophies. J. Cell Sci., 126, 5325 - 5333.
9. WO 2012/164234 A1.
10. WO 2008/133884 A2.
11. WO 2008/021210 A2.
12. Mathieu, J., Boivin, H., Meunier, D., Gaudreault, M. and Begin, P. (2001) Assessment of a disease-specific muscular impairment rating scale in myotonic dystrophy. Neurology, 56, 336 - 340.
13. Llamusi, B., Bargiela, A., Fernandez-Costa, J.M., Garcia-Lopez, A., Klima, R., Feiguin, F. and Artero, R. (2013) Muscleblind, BSF and TBPH are mislocalized in the muscle sarcomere of a Drosophila myotonic dystrophy model. Dis. Model Mech., 6, 184 - 196.
14. Laurell, H., lacovoni, J.S., Abot, A., Svec, D., Maoret, J.J., Arnal, J.F. and Kubista, M. (2012) Correction of RT-qPCR data for genomic DNA-derived signals with ValidPrime. Nucleic Acids Res., 40, e51.
15. Garcia-Lopez, A., Monferrer, L., Garcia-Alcover, I., Vicente-Crespo, M., Alvarez-Abril, M.C. and Artero, R.D. (2008) Genetic and chemical modifiers of a CUG toxicity model in Drosophila. PLoS ONE, 3, e1595.
16. Maiese, K., Chong, Z.Z., Shang, Y.C. and Wang, S. (2013) mTOR: on target for novel therapeutic strategies in the nervous system. Trends Mol. Med., 19, 51 - 60.
17. Garcia-Echeverria, C. and Sellers, W.R. (2008) Drug discovery approaches targeting the PI3K/Akt pathway in cancer. Oncogene, 27, 5511 - 5526.
18. Cheng, Y., Ren, X., Zhang, Y., Patel, R., Sharma, A., Wu, H., Robertson, G.P., Yan, L., Rubin, E. and Yang, J.M. (2011) eEF-2 kinase dictates cross-talk between autophagy and apoptosis induced by Akt Inhibition, thereby modulating cytotoxicity of novel Akt inhibitor MK-2206. Cancer Res., 71, 2654 - 2663.
19. Fernandez-Costa, J.M., Garcia-Lopez, A., Zuniga, S., Fernandez-Pedrosa, V., Felipo-Benavent, A., Mata, M., Jaka, O., Aiastui, A., Hernandez-Torres, F., Aguado, B. et al. (2013) Expanded CTG repeats trigger miRNA alterations in Drosophila that are conserved in myotonic dystrophy type 1 patients. Hum. Mol. Genet., 22, 704 - 716.
20. Yang, L., Tan, P., Zhou, W., Zhu, X., Cui, Y., Zhu, L., Feng, X., Qi, H., Zheng, J., Gu, P. et al. (2012) N-acetylcysteine protects against hypoxia mimetic-induced autophagy by targeting the HIF-1alpha pathway in retinal ganglion cells. Cell Mol. Neurobiol., 32, 1275 - 1285.
21. Shintani, T. and Klionsky, D.J. (2004) Autophagy in health and disease: a double-edged sword. Science, 306, 990 - 995.

## Claims

1. Compound, or a salt thereof, for use in the prevention and/or treatment of myotonic dystrophy type 1, wherein said compound is selected from:
a) a compound selected from the formula 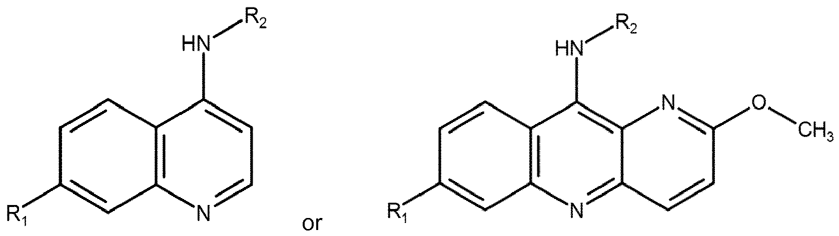 whereby
R₁ is selected from H or a halide; and
R₂ is selected from an alkyl moiety substituted with a moiety selected from an amino, alkyl amino, dialkyl amino, cycloamino or hydroxyalkyl amino moiety; or a phenolic moiety substituted with at least one moiety selected from a aminoalkyl, (alkylamino)alkyl, (dialkylamino)alkyl, (cycloamino)alkyl or (hydroxyalkylamino)alkyl moiety;
b) a compound of the formula R³SCH₂-CH(NHR⁴)-COR⁵, wherein
R³ is selected from H, -CH₂CH₂CH₂OH or -CH₂COOH;
R⁴ is selected from H or -C(=O)CH₃; and
R⁵ is selected from -OH, -OCH₃ or -NH₂; and
whereby R³ and R⁴ cannot both be H when R⁵ is -OH; or
c) a compound of the formula 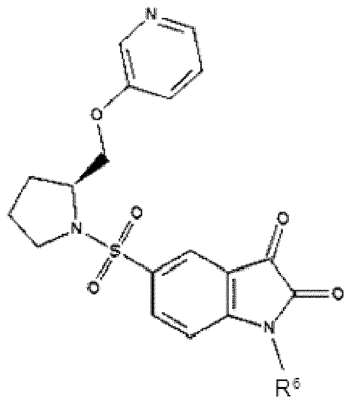 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties.

2. The compound for use according to claim 1, wherein said compound is selected from chloroquine, chloroquine phosphate, chloroquine sulfate or chloroquine hydrochloride.

3. The compound for use according to claim 1, wherein said compound is selected from *N*-acetyl L-cysteine, *N*-acetyl L-cysteine lysine salt or *N*-acetyl L-cysteine amide.

4. The compound for use according to claim 1, wherein said compound is 1-(4-methoxybenzyl)-5-({(2*S*)-2-[(3-pyridinyloxy)methyl]-1-pyrrolidinyl}sulfonyl)-1*H-*indole-2,3-dione, or a salt thereof.

5. The compound for use according to any of claims 1 to 4, wherein the myotonic dystrophy type 1 is severe congenital myotonic dystrophy type 1 or adult-onset myotonic dystrophy type 1.

6. Pharmaceutical composition for use in the prevention and/or treatment of myotonic dystrophy type 1 in a patient, wherein said pharmaceutical composition comprises:
I) a compound, or a salt thereof, selected from
a) a compound selected from the formula 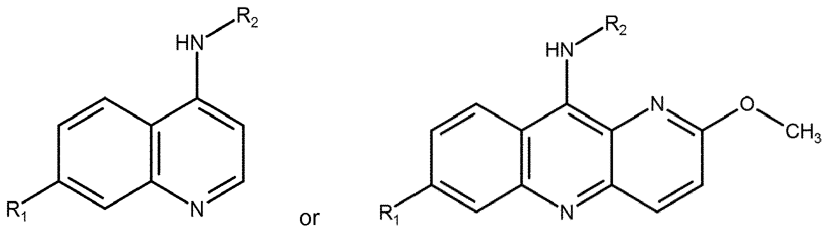 whereby
R₁ is selected from H or a halide; and
R₂ is selected from an alkyl moiety substituted with a moiety selected from an amino, alkyl amino, dialkyl amino, cycloamino or hydroxyalkyl amino moiety; or a phenolic moiety substituted with at least one moiety selected from a aminoalkyl, (alkylamino)alkyl, (dialkylamino)alkyl, (cycloamino)alkyl or (hydroxyalkylamino)alkyl moiety;
b) a compound of the formula R³SCH₂-CH(NHR⁴)-COR⁵, wherein
R³ is selected from H, -CH₂CH₂CH₂OH or -CH₂COOH;
R⁴ is selected from H or -C(=O)CH₃; and
R⁵ is selected from -OH, -OCH₃ or -NH₂; and
whereby R³ and R⁴ cannot both be H when R⁵ is -OH; or
c) a compound of the formula 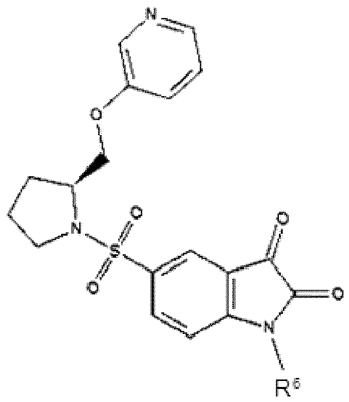 wherein R⁶ is selected from H, methyl, benzyl, 4-methoxybenzyl, 4-hydroxybenzyl, 4-(2-fluoroethoxy)benzyl, 4-bromobenzyl, 4-fluorobenzyl or 4-(methylthio)benzyl moieties; and
II) at least one excipient and/or carrier.

7. The pharmaceutical composition for use according to claim 6, wherein said compound is selected from chloroquine, chloroquine phosphate, chloroquine sulfate or chloroquine hydrochloride.

8. The pharmaceutical composition for use according to claim 6, wherein said compound is selected from N-acetyl L-cysteine, N-acetyl L-cysteine lysine salt or N-acetyl L-cysteine amide.

9. The pharmaceutical composition for use according to claim 6, wherein said compound is 1-(4-methoxybenzyl)-5-({(2S)-2-[(3-pyridinyloxy)methyl]-1-pyrrolidinyl}sulfonyl)-1*H*-indole-2,3-dione, or a salt thereof.

10. The pharmaceutical composition for use according to any of claims 6 to 9, wherein the myotonic dystrophy type 1 is severe congenital myotonic dystrophy type 1 or adult-onset myotonic dystrophy type 1.
